(19) **Europäisches Patentamt** / **European Patent Office** / **Office européen des brevets**

(11) **EP 1 538 986 B1**

(12) **EUROPEAN PATENT SPECIFICATION**

(45) Date of publication and mention
of the grant of the patent:
**15.06.2011 Bulletin 2011/24**

(21) Application number: **03767159.1**

(22) Date of filing: **01.08.2003**

(51) Int Cl.:
*A61B 8/00* (2006.01)    *G06K 9/48* (2006.01)

(86) International application number:
**PCT/US2003/024368**

(87) International publication number:
**WO 2004/012584 (12.02.2004 Gazette 2004/07)**

(54) **3D ULTRASOUND-BASED INSTRUMENT FOR NON-INVASIVE MEASUREMENT OF FLUID-FILLED AND NON FLUID-FILLED STRUCTURES**

INSTRUMENT AUF 3D-ULTRASCHALLBASIS FÜR DIE NICHTINVASIVE MESSUNG VON FLÜSSIGKEITSGEFÜLLTEN UND NICHT FLÜSSIGKEITSGEFÜLLTEN STRUKTUREN

INSTRUMENT A BASE D'ULTRASONS EN 3D DE MESURE DE STRUCTURES REMPLIES OU NON DE LIQUIDES

(84) Designated Contracting States:
**AT BE BG CH CY CZ DE DK EE ES FI FR GB GR HU IE IT LI LU MC NL PT RO SE SI SK TR**

(30) Priority: **02.08.2002 US 400624 P**
**05.11.2002 US 423881 P**
**12.05.2003 US 470525 P**
**20.05.2003 US 443126**
**31.07.2003 US 633186**

(43) Date of publication of application:
**15.06.2005 Bulletin 2005/24**

(73) Proprietor: **Verathon Inc.**
**Bothell, WA 98021 (US)**

(72) Inventors:
• **CHALANA, Vikram**
**Mill Creek, WA 98027 (US)**

• **DUDYCHA, Stephen**
**Bothell, WA 98011 (CA)**
• **MCMORROW, Gerald**
**Mill Creek, WA 98027 (US)**

(74) Representative: **Land, Addick Adrianus Gosling et al**
**Arnold & Siedsma**
**Sweelinckplein 1**
**2517 GK Den Haag (NL)**

(56) References cited:
**EP-A- 1 030 187        EP-A- 1 076 318**
**US-A- 5 148 809        US-A- 5 465 721**
**US-A- 5 605 155        US-A- 5 993 390**
**US-A1- 2002 102 023    US-B1- 6 375 616**
**US-B1- 6 610 013**

**Description**

**FIELD OF THE INVENTION**

This invention pertains to the field of image enhancement and analysis, particularly to electromagnetic and non-electromagnetic acquired images, to ultrasound-based non-invasive obstetric measurements, and to the field of medical imaging, particularly to measuring the size and shape of organs from three-dimensional images.

**BACKGROUND OF THE INVENTION**

Measurement of the amount of Amniotic Fluid (AF) volume is critical for assessing the kidney and lung function of a fetus and also for assessing the placental function of the mother. Amniotic fluid volume is also a key measure to diagnose conditions such as polyhydramnios (too much AF) and oligohydramnios (too little AF). Polyhydramnios and oligohydramnios are diagnosed in about 7-8% of all pregnancies and these conditions are of concern because they may lead to birth defects or to delivery complications. The amniotic fluid volume is also one of the important components of the fetal biophysical profile, a major indicator of fetal well-being.

The currently practiced and accepted method of quantitatively estimating the AF volume is from two-dimensional (2D) ultrasound images. The most commonly used measure is known as the use of the amniotic fluid index (AFI). AFI is the sum of vertical lengths of the largest AF pockets in each of the 4 quadrants. The four quadrants are defined by the umbilicus (the navel) and the linea nigra (the vertical mid-line of the abdomen). The transducer head is placed on the maternal abdomen along the longitudinal axis with the patient in the supine position. This measure was first proposed by Phelan et al (Phelan JP, Smith CV, Broussard P, Small M., "Amniotic fluid volume assessment with the four-quadrant technique at 36-42 weeks' gestation," J Reprod Med Jul; 32(7): 540-2, 1987) and then recorded for a large normal population over time by Moore and Cayle (Moore TR, Cayle JE. "The amniotic fluid index in normal human pregnancy," Am J Obstet Gynecol May; 162(5): 1168-73, 1990).

Even though the AFI measure is routinely used, studies have shown a very poor correlation of the AFI with the true AF volume (Sepulveda W, Flack NJ, Fisk NM., "Direct volume measurement at midtrimester amnioinfusion in relation to ultrasonographic indexes of amniotic fluid volume," Am J Obstet Gynecol Apr; 170(4): 1160-3, 1994). The correlation coefficient was found to be as low as 0.55, even for experienced sonographers. The use of vertical diameter only and the use of only one pocket in each quadrant are two reasons why the AFI is not a very good measure of AF Volume (AFV).

Some of the other methods that have been used to estimate AF volume include:

**Dye dilution technique.** This is an invasive method where a dye is injected into the AF during amniocentesis and the final concentration of dye is measured from a sample of AF removed after several minutes. This technique is the accepted gold standard for AF volume measurement; however, it is an invasive and cumbersome method and is not routinely used.

**Subjective interpretation from ultrasound images.** This technique is obviously dependent on observer experience and has not been found to be very good or consistent at diagnosing oligo- or poly-hydramnios.

**Vertical length of the largest single cord-free pocket.** This is an earlier variation of the AFI where the diameter of only one pocket is measured to estimate the AF volume.

**Two-diameter areas of the largest AF pockets in the four quadrants.** This is similar to the AFI; however, in this case, two diameters are measured instead of only one for the largest pocket. This two diameter area has been recently shown to be better than AFI or the single pocket measurement in identifying oligohydramnios (Magann EF, Perry KG Jr, Chauhan SP, Anfanger PJ, Whitworth NS, Morrison JC., "The accuracy of ultrasound evaluation of amniotic fluid volume in singleton pregnancies: the effect of operator experience and ultrasound interpretative technique," J Clin Ultrasound, Jun; 25(5):249-53, 1997).

The measurement of various anatomical structures using computational constructs are described, for example, in U.S patent 6,346,124 to Geiser, et al. (Autonomous Boundary Detection System For Echocardiographic Images). Similarly, the measurement of bladder structures are covered in U.S. patent 6,213,949 to Ganguly, et al. (System For Estimating Bladder Volume) and U.S. patent to 5,235,985 to McMorrow, et al., (Automatic Bladder Scanning Apparatus). The measurement of fetal head structures is described in U.S. patent 5,605,155 to Chalana, et al., (Ultrasound System For Automatically Measuring Fetal Head Size). The measurement of fetal weight is described in U.S. patent 6,375,616 to Soferman, et al. (Automatic Fetal Weight Determination).

Pertaining to ultrasound-based determination of amniotic fluid volumes, Segiv et al. (in Segiv C, Akselrod S, Tepper R., "Application of a semiautomatic boundary detection algorithm for the assessment of amniotic fluid quantity from ultrasound images." Ultrasound Med Biol, May; 25(4): 515-26, 1999) describe a method for amniotic fluid segmentation from 2D images. However, the Segiv et al. method is interactive in nature and the identification of amniotic fluid volume is very observer dependent. Moreover, the system described is not a dedicated device for amniotic fluid volume assessment.

Grover et al. (Grover J, Mentakis EA, Ross MG, "Three-dimensional method for determination of amniotic fluid volume in intrauterine pockets." Obstet Gynecol, Dec; 90(6): 1007-10, 1997) describe the use of a urinary bladder volume instrument for amniotic fluid volume measurement. The Grover et al. method makes use of the bladder volume instrument without any modifications and uses shape and other anatomical assumptions specific to the bladder that do not generalize to amniotic fluid pockets. Amniotic fluid pockets having shapes not consistent with the Grover et al. bladder model introduces analytical errors. Moreover, the bladder volume instrument does not allow for the possibility of more than one amniotic fluid pocket in one image scan. Therefore, the amniotic fluid volume measurements made by the Grover et al. system may not be correct or accurate.

As relating to the measurement of the size, shape, and internal volumes of specific organs using ultrasound segmentation analysis from three-dimensional ultrasound images, various methods have been used or proposed. Various kinds of methods have been used or proposed in the literature to measure the size, shape, and internal volumes of specific organs using ultrasound segmentation analysis from three-dimensional ultrasound images. Examples of body structures undergoing segmentation analysis include heart chambers, particularly cardiac chambers (left and right ventricles, left and right atriums), the prostate, the bladder, and amniotic sacs.

The measurement of heart chambers is described, for example in U.S patent 6,346,124 to Geiser, et al. (Autonomous Boundary Detection System For Echocardiographic Images). Similarly, the measurement of bladder structures are covered in U.S. patent 6,213,949 to Ganguly, et al. (System For Estimating Bladder Volume) and U.S. patent to 5,235,985 to McMorrow, et al., (Automatic Bladder Scanning Apparatus). The measurement of fetal head structures is described in U.S. patent 5,605,155 to Chalana, et al., (Ultrasound System For Automatically Measuring Fetal Head Size). The measurement of fetal weight is described in U.S. patent 6,375,616 to Soferman, et al. (Automatic Fetal Weight Determination).

Most of these techniques are not ideal in that they cannot be generalized beyond the application for which they were designed. They use shape or size assumptions that are not necessarily satisfied for different applications. An attempt to make a generalized model is described in U.S. patent 5,588,435 to Weng, et al. (System And Method For Automatic Measurement Of Body Structures) but is limited in its application due to method complexity and operational robustness.

The techniques commonly employed in segmentation use active contour modeling and live wire methods to delineate an object shape within electronic-based images, and partial differential equation (PDE) based processing of image signals to reduce background noise speckle. These three methods have their advantages and disadvantages. For example, the active contour modeling method or "snakes" is a region-based boundary method and requires specific shape assumptions and the use of deformable models to work properly (M. Kass, A. Witkin, D. Terzopolous, "Snakes : Active Contour Models, "International Journal of Computer Vision, pp. 321-331, 1988). However, "snake" methods require an initial contour that needs to be very close to the final desired boundary. In addition, most of these methods are iterative in nature and labor intensive.

U.S. patent application published under number US2002/0102023 A1 discloses an ultrasonic diagnostic device including an automatic contour extracting unit that contains: an initial contour extracting unit for roughly extracting an initial contour of an object to be examined from an ultrasound image by performing a predetermined operation (such as equalization, binarization and degeneration) on the ultrasound image; and a dynamic contour extracting unit for accurately extracting a final contour of the object by using the extracted initial contour as an initial value and by applying an active contour model, such as the SNAKES model, to the object within the ultrasound image.

Live wire methods, championed by A. X. Falacao et al. in "User-steered Image Segmentation Paradigms: Live wire and Live Lane," (A.X. Falacao, J.K. Udupa, S. Samarasekara, and S. Sharma, Graphical Models and Image Processing, 60,233-26-, 1998) and E.W. Dijkstra, in "A note on two problems in connection with graphs," (Numerical Math, vol. 1, pp. 269-271, 1959), requires a user to actively provide a starting point and an ending point via a mouse, pointer, or equivalent delineation means. Live wire is an interactive segmentation method which uses a minimum cost path between the last clicked user point to the current mouse clicked location. The live wire methodology of Falacao is labor intensive since it requires an iterative approach and generates a series of mouse delineating end-points. Furthermore, live wire methods are limited in accuracy because the simplest optimal path contour connecting any two mouse determined end-points is not sufficient in ultrasound images that are prone to mouse pointer backtracking. That is, the "user-steering" methods of Falacao require that the user backtrack the mouse whenever the drawn contour line has lost track of the object boundary as presented on the ultrasound image.

Most ultrasound processing methods involve some pre-processing techniques to reduce speckle noise and enhance images, such as median filtering methods and PDE-based image processing. PDE-based methods have the added advantages of preserving edges while enhancing images (see P. Perona and J. Malik, "Scale-space and edge detection using aniostropic diffusion," IEEE Trans. Pattern Analysis and Machine Intelligence, vol. 12, July 1990, pp. 629 - 639; J.A. Sethian, Level Set Methods and Fast Marching Methods, 2nd Edition, Cambridge University Press, 1999; S. Osher and L.I. Rudin, "Feature-oriented image enhancement using shock filters," SIAM Journal of Numerical Analysis, vol. 27, pp. 919-940, August 1990; and U.S. patent 5,644,513 to Rudin, et al. "System Incorporating Feature-Oriented Signal Enhancement Using Shock Filters"). These methods, especially shock filters for image sharpening or blind deconvolution, have been used reducing noise speckle for computerized-tomography (CT), magnetic resonance (MR), and positron emission-tomography (PET) images. However, shock filters implemented with unoptimized algorithms have been found deficient in that the unoptimized algorithms increase or enhance noise speckle, thereby degrading the CT, MR, and PET images.

In ultrasound images, chamber-like structures (for example: bladder, heart left ventricle, prostate, amniotic sac) are further prone to image degradation due to ultrasound echoes reflecting from other surfaces parallel to do organ wall boundary. The echoes from the parallel surfaces present as bright regions overlapping with the dark chamber boundary walls. Thus, ultrasound-echoing overlaps creates missing lateral boundaries of the organ walls. Thereby, making it difficult to accurately determine boundary wall locations where segmentation lines need to be drawn.

None of the currently used methods for AF volume estimation are ideal. Therefore, there is a need for better, non-invasive, and easier ways to accurately measure amniotic fluid volume. Furthermore, there is a need for a generalized image analysis method to accurately measure the size, shape, and volumes of fluid-filled and non-fluid filled organs from analysis of one-dimensional (1D), two-dimensional (2D), and three-dimensional (3D) images from ultrasound, CT, MR, and PET procedures. The generalized image analysis method requires optimal algorithms to minimize background noise speckle and maximize structure segmentation or delineation by minimizing the creation of missed lateral boundaries of organ structures. The generalized image analysis method needs to be broadly adaptable and simple to implement.

## SUMMARY OF THE INVENTION

The invention is as defined by the amended set of claims.

The preferred form of the invention is a three dimensional (3D) ultrasound-based system and method having a plurality of automated processes optimized to robustly locate and measure the volume of amniotic fluid in a uterus without resorting to pre-conceived models of the shapes of amniotic fluid pockets in ultrasound images. The automated process uses a plurality of algorithms in a sequence that includes steps for image enhancement, segmentation, and polishing.

A hand-held 3D ultrasound device is used to image the uterus trans-abdominally. The user moves the device around on the maternal abdomen and, using 2D image processing to locate the amniotic fluid areas, the device gives feedback to the user about where to acquire the 3D image data sets. The user acquires one or more 3D image data sets covering all of the amniotic fluid in the uterus and the data sets are then stored in the device or transferred to a host computer.

The 3D datasets are then subjected to a 3D analysis process, the 3D analysis process preferably having a plurality of processes to calculate and the total volume of amniotic fluid in the uterus. The plurality of processes is either implemented on the device itself or is implemented on the host computer. Alternatively, the plurality of processes can also be implemented on a server or other computer to which the 3D ultrasound data sets are transferred.

In one preferred 3D analysis process, each 2D image in the 3D dataset is first enhanced using non-linear filters by an image pre-filtering step. The image pre-filtering step includes an image-smoothing step to reduce image noise followed by an image-sharpening step to obtain maximum contrast between organ wall boundaries.

A second process includes subjecting the resulting image of the first process to a location method to identify initial edge points between amniotic fluid and other fetal or maternal structures. The location method automatically determines the leading and trailing regions of wall locations along an A-mode one-dimensional scan line.

A third process includes subjecting the image of the first process to an intensity-based segmentation process where dark pixels (representing fluid) are automatically separated from bright pixels (representing tissue and other structures).

In a fourth process, the images resulting from the second and third step are combined to result in a single image representing likely amniotic fluid regions.

In a fifth process, the combined image is cleaned to make the output image smooth and to remove extraneous structures such as the fetal head and the fetal bladder.

A sixth process includes placing boundary line contours on each 2D image. Thereafter, the method then calculates the total 3D volume of amniotic fluid.

The system and method further provides an automatic method to detect and correct for any contribution the fetal head provides to the amniotic fluid volume.

Another preferred embodiment of the invention is a method having a plurality of segmentation algorithms and steps optimized to robustly locate and measure the volume of a fluid filled or non-fluid filled structure or organ from primary images obtained from instruments probing with or receiving and processing electromagnetic and non-electromagnetic radiation including ultrasound, CT, MR, PET, and radioisotopes used in nuclear medicine procedures that produce a plurality of 1D, 2D, or 3D images. The method is applied to 1D scans to obtain resultant 2D primary images. Each 2D primary image is made visually enhanced by the plurality of segmentation algorithms and steps of the method. The method then includes the creation and placement of accurately drawn boundary line contours that more clearly delineate or segment the structure walls within the 2D image. Upon placing the boundary line contours, the method calculates the volumes for each delineated structure or organ presented in each primary image. Then, as an aggregate, the method provides the ability to calculate the 2D area and the 3D volume of the structure or organ, fluid filled or non-fluid filled, from the summation of each 2D image.

The method employs subjecting each primary image to a pre-filtering step using non-linear filters to enhance the image. The pre-filtering step includes an image-smoothing step to obtain maximum contrast between organ wall boundaries followed by an image-sharpening step to establish image enhancements. The pre-filtering step has a first algorithm and a second algorithm, each algorithm having a partial differential equation (PDE) formula in non-linear form. The first PDE algorithm serves as a heat filter, and the second PDE algorithm serves as a shock filter. The sequence of image enhancement is application of the heat filter followed by application of the shock filter. Each image is subjected to a combination of the heat and shock filters to reduce each image's signal noise and to sharpen each image signal while at the same time preserving signal discontinuities. Each primary image subjected to the heat and shock prefiltering algorithms is formed into a second image having a clearer demarcation along the boundary of the structure or organ walls.

Each second image is then processed to determine where to locate initial edge points by either of two locating methods. A first locating method is a manual, user-deployed radial point method. The radial point method is comprised of lines that are drawn in substantially 30-degree increments for establishing where maximum spatial gradients exist during transitioning between light and dark areas. A second locating method to set initial boundary edge points is an automatic method to determine the leading and trailing regions of wall locations along an A-mode first dimension (1D) scan line that is regulated by a hysteresis threshold value.

An optimal pathway to connect between the initial edge points is then determined using a third algorithm to determine an optimal pathway. The optimal pathway between any two points is one defined to have the least cost, where the least cost represents the sum of three sub-equations; namely, the edge distance cost, the path direction cost, and the previous contour distance cost. The optimal pathway represents an automatically implemented way to draw contour boundary lines along wall structure using the automatically selected initial edge points from the second locating method or as manually selected by the radial method. The advantages compared to the manual live wire methodology is fewer initial points are needed to establish boundary contours, the minimum cost-optimal pathway algorithm is more robust, and does not require manual interactive mouse pointer setting and reselecting.

After the boundaries of each structure or organ are determined in each second image, the 2D area and 3D volume of the organ or structure is determined. For fluid-filled organs, this includes but is not limited to bladders, amniotic sacs, prostates, cardiac chambers (left and right ventricles, left and right atriums), ovarian cysts, arteries, and veins. Similarly, non-fluid containing organs or structures includes but is not limited to kidneys, kidney stones, gall bladders, gall bladder stones, and ovaries.

Finally, the method may be done remotely using a web server to analyze scan data sent from ultrasound, CT, MR, and PET devices over intranets or internets where the device architecture permits image processing on the server-side of the invention.

**BRIEF DESCRIPTION OF THE DRAWINGS**

FIGURE 1 is a side view of a microprocessor-controlled, hand-held ultrasound transceiver;

FIGURE 2A is a is depiction of the hand-held transceiver in use for scanning a patient;

FIGURE 2B is a perspective view of the hand-held transceiver device sitting in a communication cradle;

FIGURE 3 depicts a schematic view of a plurality of transceivers in connection with a server;

FIGURE 4 depicts a schematic view of a plurality of transceivers in connection with a server over a network;

FIGURE 5A a graphical representation of a plurality of scan lines forming a single scan plane;

FIGURE 5B is a graphical representation of a plurality of scanplanes forming a three-dimensional array having a substantially conic shape;

FIGURE 6 is a depiction of the hand-held transceiver placed laterally on a patient trans-abdominally to transmit ultrasound and receive ultrasound echoes for processing to determine amniotic fluid volumes;

FIGURE 7 shows a block diagram overview of the two-dimensional and three-dimensional Input, Image Enhancement, Intensity-Based Segmentation, Edge-Based Segmentation, Combine, Polish , Output, and Compute algorithms to visualize and determine the volume or area of amniotic fluid;

FIGURE 8A depicts the sub-algorithms of Image Enhancement;

FIGURE 8B depicts the sub-algorithms of Intensity-Based Segmentation;

FIGURE 8C depicts the sub-algorithms of Edge-Based Segmentation;

FIGURE 8D depicts the sub-algorithms of the Polish algorithm, including Close, Open, Remove Deep Regions, and Remove Fetal Head Regions;

FIGURE 8E depicts the sub-algorithms of the Remove Fetal Head Regions sub-algorithm;

FIGURE 8F depicts the sub-algorithms of the Hough Transform sub-algorithm;

FIGURE 9 depicts the operation of a circular Hough transform algorithm;

FIGURE 10 shows results of sequentially applying the algorithm steps on a sample image;

FIGURE 11 illustrates a set of intermediate images of the fetal head detection process;

FIGURE 12 presents a 4-panel series of sonographer amniotic fluid pocket outlines and the algorithm output amniotic fluid pocket outlines;

Figure 13 is an overview of the segmentation algorithm method;

Figure 14 is an algorithm processing on a real bladder image presented in a five-panel series;

Figure 15 is a three panel series of 2D images smoothed with varying heat filter algorithm iterations;

Figure 16A is a graph showing the effects of a shock-filtering algorithm of a blurred signal;

Figure 16B is a graph showing the effects of heat and shock filtering algorithms of a blurred noisy signal;

Figure 17 is a pixel-processing algorithm for finding the direction cost;

Figure 18 is a 24 panel series of segmentation algorithms applied to 24 image planes of a bladder phantom, each image plane showing the resultant boundary contours;

Figure 19 is a 12 panel series of segmentation algorithms applied to a first human bladder scanned with 12 images planes, each image plane showing the resultant boundary contours; and

Figure 20 is a 12 panel series of segmentation algorithms applied to a second human bladder scanned with 12 image planes, each image plane showing the resultant boundary contours.

## DETAILED DESCRIPTION OF THE PREFERRED EMBODIMENT

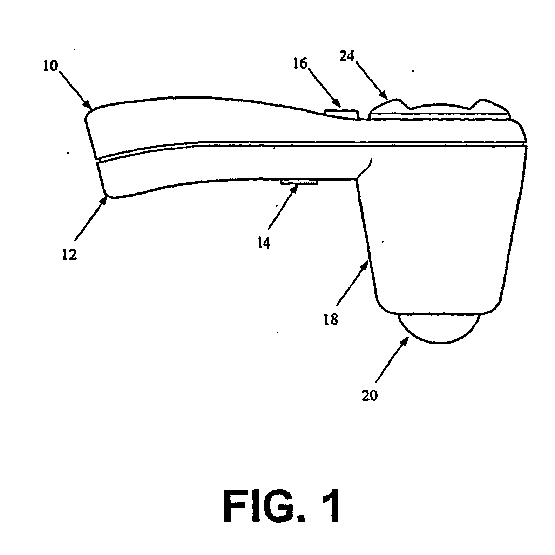

The preferred portable embodiment of the ultrasound transceiver of the amniotic fluid volume measuring system are shown in FIGURES 1-4. The transceiver 10 includes a handle 12 having a trigger 14 and a top button 16, a transceiver housing 18 attached to the handle 12, and a transceiver dome 20. A display 24 for user interaction is attached to the transceiver housing 18 at an end opposite the transceiver dome 20. Housed within the transceiver 10 is a single element transducer (not shown) that converts ultrasound waves to electrical signals. The transceiver 10 is held in position against the body of a patient by a user for image acquisition and signal processing. In operation, the transceiver 10 transmits a radio frequency ultrasound signal at substantially 3.7 MHz to the body and then receives a returning echo signal. To accommodate different patients having a variable range of obesity, the transceiver 10 can be adjusted to transmit a range of probing ultrasound energy from approximately 2 MHz to approximately 10 MHz radio frequencies.

The top button 16 selects for different acquisition volumes. The transceiver is controlled by a microprocessor and software associated with the microprocessor and a digital signal processor of a computer system. As used in this invention, the term "computer system" broadly comprises any microprocessor-based or other computer system capable of executing operating instructions and manipulating data, and is not limited to a traditional desktop or notebook computer. The display 24 presents alphanumeric or graphic data indicating the proper or optimal positioning of the transceiver 10 for initiating a series of scans. A suitable transceiver is the DCD372 made by Diagnostic Ultrasound. In alternate embodiments, the two- or three-dimensional image of a scan plane may be presented in the display 24.

Although the preferred ultrasound transceiver is described above, other transceivers may also be used. For example, the transceiver need not be battery-operated or otherwise portable, need not have a top-mounted display 24, and may include many other features or differences. The display 24 may be a liquid crystal display (LCD), a light emitting diode (LED), a cathode ray tube (CRT), or any suitable display capable of presenting alphanumeric data or graphic images.

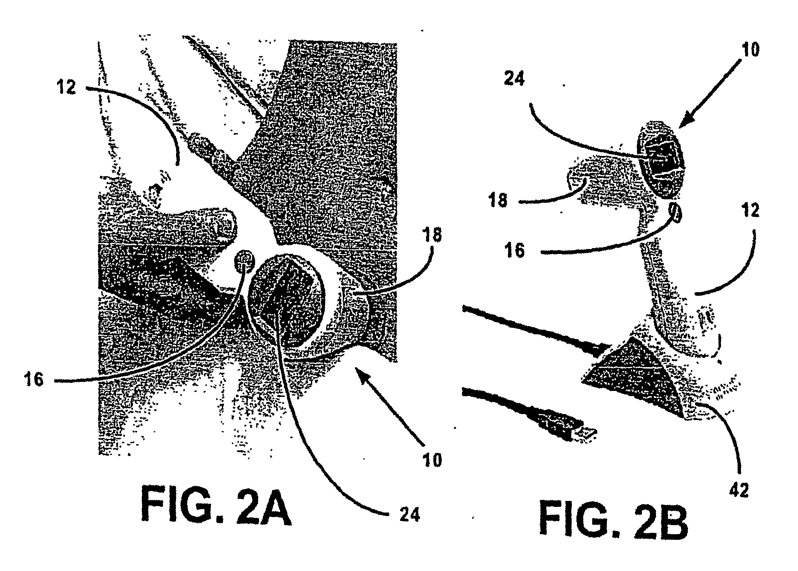

FIGURE 2A is a photograph of the hand-held transceiver 10 for scanning a patient. The transceiver 10 is then positioned over the patient's abdomen by a user holding the handle 12 to place the transceiver housing 18 against the patient's abdomen. The top button 16 is centrally located on the handle 12. Once optimally positioned over the abdomen for scanning, the transceiver 10 transmits an ultrasound signal at substantially 3.7 MHz into the uterus. The transceiver 10 receives a return ultrasound echo signal emanating from the uterus and presents it on the display 24.

FIGURE 2B is a perspective view of the hand-held transceiver device sitting in a communication cradle. The transceiver 10 sits in a communication cradle 42 via the handle 12. This cradle can be connected to a standard USB port of any personal computer, enabling all the data on the device to be transferred to the computer and enabling new programs to be transferred into the device from the computer.

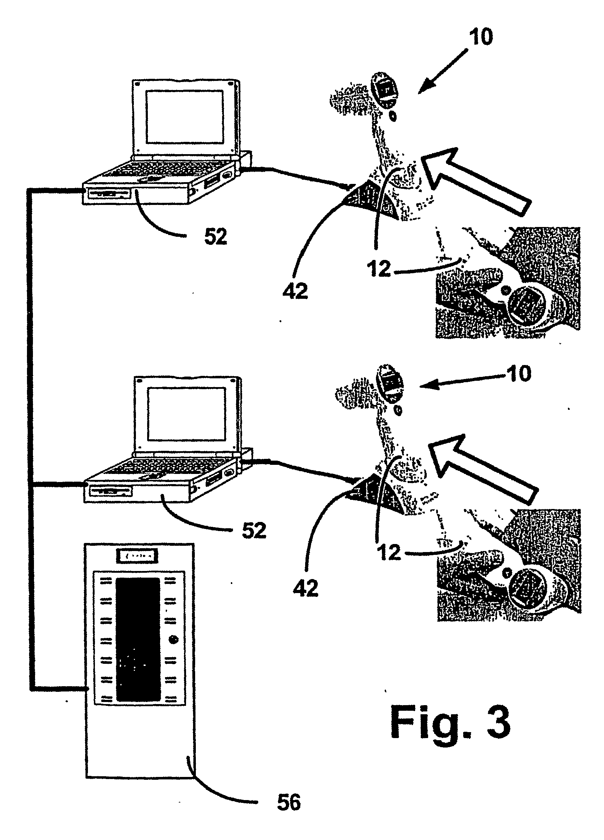

FIGURE 3 depicts a schematic view of a plurality of transceivers in connection with a server. FIGURE 3, by example, depicts each transceiver 10 being used to send probing ultrasound radiation to a uterus of a patient and to subsequently retrieve ultrasound echoes returning from the uterus, convert the ultrasound echoes into digital echo signals, store the digital echo signals, and process the digital echo signals by algorithms of the invention. A user holds the transceiver 10 by the handle 12 to send probing ultrasound signals and to receive incoming ultrasound echoes. The transceiver 10 is placed in the communication cradle 42 that is in signal communication with a computer 52, and operates as an amniotic fluid volume measuring system. Two amniotic fluid volume-measuring systems are depicted as representative though fewer or more systems may be used. As used in this invention, a "server" can be any computer software or hardware that responds to requests or issues commands to or from a client. Likewise, the server may be accessible by one or more client computers via the Internet, or may be in communication over a LAN or other network.

Each amniotic fluid volume measuring systems includes the transceiver 10 for acquiring data from a patient. The transceiver 10 is placed in the cradle 52 to establish signal communication with the computer 52. Signal communication as illustrated is by a wired connection from the cradle 42 to the computer 52. Signal communication between the transceiver 10 and the computer 52 may also be by wireless means, for example, infrared signals or radio frequency signals. The wireless means of signal communication may occur between the cradle 42 and the computer 52, the transceiver 10 and the computer 52, or the transceiver 10 and the cradle 42.

A preferred first embodiment of the amniotic fluid volume measuring system includes each transceiver 10 being separately used on a patient and sending signals proportionate to the received and acquired ultrasound echoes to the computer 52 for storage. Residing in each computer 52 are imaging programs having instructions to prepare and analyze a plurality of one dimensional (1D) images from the stored signals and transforms the plurality of 1D images into the plurality of 2D scanplanes. The imaging programs also present 3D renderings from the plurality of 2D scanplanes. Also residing in each computer 52 are instructions to perform the additional ultrasound image enhancement procedures, including instructions to implement the image processing algorithms.

A preferred second embodiment of the amniotic fluid volume measuring system is similar to the first embodiment, but the imaging programs and the instructions to perform the additional ultrasound enhancement procedures are located on the server 56. Each computer 52 from each amniotic fluid volume measuring system receives the acquired signals from the transceiver 10 via the cradle 51 and stores the signals in the memory of the computer 52. The computer 52 subsequently retrieves the imaging programs and the instructions to perform the additional ultrasound enhancement procedures from the server 56. Thereafter, each computer 52 prepares the 1D images, 2D images, 3D renderings, and enhanced images from the retrieved imaging and ultrasound enhancement procedures. Results from the data analysis procedures are sent to the server 56 for storage.

A preferred third embodiment of the amniotic fluid volume measuring system is similar to the first and second embodiments, but the imaging programs and the instructions to perform the additional ultrasound enhancement procedures are located on the server 56 and executed on the server 56. Each computer 52 from each amniotic fluid volume measuring system receives the acquired signals from the transceiver 10 and via the cradle 51 sends the acquired signals in the memory of the computer 52. The computer 52 subsequently sends the stored signals to the server 56. In the server 56, the imaging programs and the instructions to perform the additional ultrasound enhancement procedures are executed to prepare the 1D images, 2D images, 3D renderings, and enhanced images from the server 56 stored signals. Results from the data analysis procedures are kept on the server 56, or alternatively, sent to the computer 52.

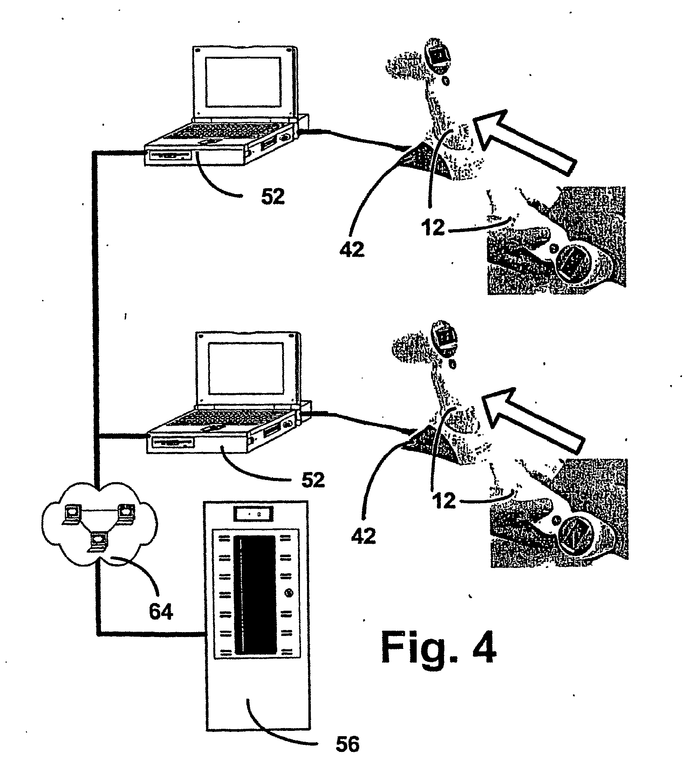

FIGURE 4 is a schematic view of a plurality of amniotic fluid measuring systems connected to a server over the Internet or other network 64. FIGURE 4 represents any of the first, second, or third embodiments of the invention advantageously deployed to other servers and computer systems through connections via the network.

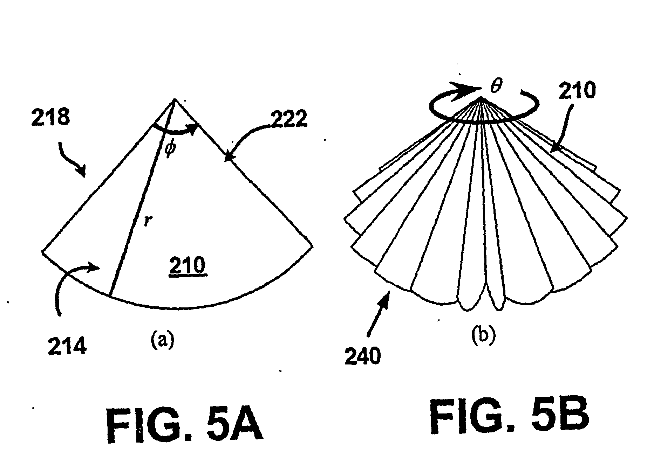

FIGURE 5A a graphical representation of a plurality of scan lines forming a single scan plane. FIGURE 5A illustrates how ultrasound signals are used to make analyzable images, more specifically how a series of one-dimensional (1D) scanlines are used to produce a two-dimensional (2D) image. The 1D and 2D operational aspects of the single element tranducer housed in the transceiver 10 is seen as it rotates mechanically about an angle φ. A scanline 214 of length r migrates between a first limiting position 218 and a second limiting position 222 as determined by the value of the angle φ, creating a fan-like 2D scanplane 210. In one preferred form, the transceiver 10 operates substantially at 3.7 MHz frequency and creates an approximately 18 cm deep scan line 214 and migrates within the angle φ having an angle of approximately 0.027 radians. A first motor tilts the transducer approximately 60° clockwise and then counterclockwise forming the fan-like 2D scanplane presenting an approximate 120° 2D sector image. A plurality of scanlines, each scanline substantially equivalent to scanline 214 is recorded, between the first limiting position 218 and the second limiting position 222 formed by the unique tilt angle φ. The plurality of scanlines between the two extremes forms a scanplane 210. In the preferred embodiment, each scanplane contains 77 scan lines, although the number of lines can vary within the scope of this invention. The tilt angle φ sweeps through angles approximately between -60° and +60° for a total arc of approximately 120°.

FIGURE 5B is a graphical representation of a plurality of scanplanes forming a three-dimensional array (3D) 240 having a substantially conic shape. FIGURE 5B illustrates how a 3D rendering is obtained from the plurality of 2D scanplanes. Within each scanplane 210 are the plurality of scanlines, each scanline equivalent to the scanline 214 and sharing a common rotational angle θ. In the preferred embodiment, each scanplane contains 77 scan lines, although the number of lines can vary within the scope of this invention. Each 2D sector image scanplane 210 with tilt angle φ and range r (equivalent to the scanline 214) collectively forms a 3D conic array 240 with rotation angle θ. After gathering the 2D sector image, a second motor rotates the transducer between 3.75° or 7.5° to gather the next 120° sector image. This process is repeated until the transducer is rotated through 180°, resulting in the cone-shaped 3D conic array 240 data set with 24 planes rotationally assembled in the preferred embodiment. The conic array could have fewer or more planes rotationally assembled. For example, preferred alternate embodiments of the conic array could include at least two scanplanes, or a range of scanplanes from 2 to 48 scanplanes. The upper range of the scanplanes can be greater than 48 scanplanes. The tilt angle φ indicates the tilt of the scanline from the centerline in 2D sector image, and the rotation angle θ, identifies the particular rotation plane the sector image lies in. Therefore, any point in this 3D data set can be isolated using coordinates expressed as three parameters, $P(r,\phi,\theta)$.

As the scanlines are transmitted and received, the returning echoes are interpreted as analog electrical signals by a transducer, converted to digital signals by an analog-to-digital converter, and conveyed to the digital signal processor of the computer system for storage and analysis to determine the locations of the amniotic fluid walls. The computer system is representationally depicted in FIGURES 3 and 4 and includes a microprocessor, random access memory (RAM), or other memory for storing processing instructions and data generated by the transceiver 10.

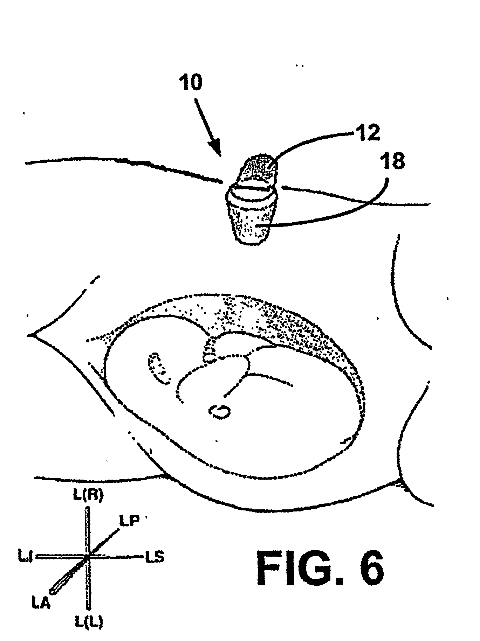

FIGURE 6 is a depiction of the hand-held transceiver placed on a patient trans-abdominally to transmit probing ultrasound and receive ultrasound echoes for processing to determine amniotic fluid volumes. The transceiver 10 is held by the handle 12 to position over a patient to measure the volume of amniotic fluid in an amniotic sac over a baby. A plurality of axes for describing the orientation of the baby, the amniotic sac, and mother is illustrated. The plurality of axes includes a vertical axis depicted on the line L(R) - L(L) for left and right orientations, a horizontal axis LI - LS for inferior and superior orientations, and a depth axis LA - LP for anterior and posterior orientations.

FIGURE 6 is representative of a preferred data acquisition protocol used for amniotic fluid volume determination. In this protocol, the transceiver 10 is the hand-held 3D ultrasound device (for example, model DCD372 from Diagnostic Ultrasound) and is used to image the uterus trans-abdominally. Initially during the targeting phase, the patient is in a supine position and the device is operated in a 2D continuous acquisition mode. A 2D continuous mode is where the data is continuously acquired in 2D and presented as a scanplane similar to the scanplane 210 on the display 24 while an operator physically moves the transceiver 10. An operator moves the transceiver 10 around on the maternal abdomen and the presses the trigger 14 of the transceiver 10 and continuously acquires real-time feedback presented in 2D on the display 24. Amniotic fluid, where present, visually appears as dark regions along with an alphanumeric indication of amniotic fluid area (for example, in cm$^2$) on the display 24. Based on this real-time information in terms of the relative position of the transceiver 10 to the fetus, the operator decides which side of the uterus has more amniotic fluid by the presentation on the display 24. The side having more amniotic fluid presents as regions having larger darker regions on the display 24. Accordingly, the side displaying a large dark region registers greater alphanumeric area while the side with less fluid shows displays smaller dark regions and proportionately registers smaller alphanumeric area on the display 24. While amniotic fluid is present throughout the uterus, its distribution in the uterus depends upon where and how the fetus is positioned within the uterus. There is usually less amniotic fluid around the fetus's spine and back and more amniotic fluid in front of its abdomen and around the limbs.

Based on fetal position information acquired from data gathered under continuous acquisition mode, the patient is placed in a lateral recumbent position such that the fetus is displaced towards the ground creating a large pocket of amniotic fluid close to abdominal surface where the transceiver 10 can be placed as shown in FIGURE 6. For example, if large fluid pockets are found on the right side of the patient, the patient is asked to turn with the left side down and if large fluid pockets are found on the left side, the patient is asked to turn with the right side down.

After the patient has been placed in the desired position, the transceiver 10 is again operated in the 2D continuous acquisition mode and is moved around on the lateral surface of the patient's abdomen. The operator finds the location that shows the largest amniotic fluid area based on acquiring the largest dark region imaged and the largest alphanumeric value displayed on the display 24. At the lateral abdominal location providing the largest dark region, the transceiver 10 is held in a fixed position, the trigger 14 is released to acquire a 3D image comprising a set of arrayed scanplanes. The 3D image presents a rotational array of the scanplanes 210 similar to the 3D array 240. In a preferred alternate data acquisition protocol, the operator can reposition the transceiver 10 to a different abdominal location to acquire new 3D images comprised of different scanplane arrays similar to the 3D array 240. Multiple scan cones obtained from different lateral positions provide the operator the ability to verify amniotic fluid imaging and measurement. In the case of a single image cone being too small to accommodate a large AFV measurement, obtaining multiple 3D array 240 image cones ensures that the total volume of large AFV regions is determined. Multiple 3D images may also be acquired by pressing the top bottom 16 to select multiple conic arrays similar to the 3D array 240.

Depending on the position of the fetus relative to the location of the transceiver 10, a single image scan may present an underestimated volume of AFV due to amniotic fluid pockets that remain hidden behind the limbs of the fetus. The hidden amniotic fluid pockets present as unquantifiable shadow-regions.

To guard against underestimating AFV, repeated positioning the transceiver 10 and rescanning can be done to obtain more than one ultrasound view to maximize detection of amniotic fluid pockets. Repositioning and rescanning provides multiple views as a plurality of the 3D arrays 240 images cones. Acquiring multiple images cones improves the probability of obtaining initial estimates of AFV that otherwise could remain undetected and un-quantified in a single scan.

In an alternative scan protocol, the user determines and scans at only one location on the entire abdomen that shows the maximum amniotic fluid area while the patient is the supine position. As before, when the user presses the top button 16, 2D scanplane images equivalent to the scanplane 210 are continuously acquired and the amniotic fluid area on every image is automatically computed. The user selects one location that shows the maximum amniotic fluid area. At this location, as the user releases the scan button, a full 3D data cone is acquired and stored in the device's memory.

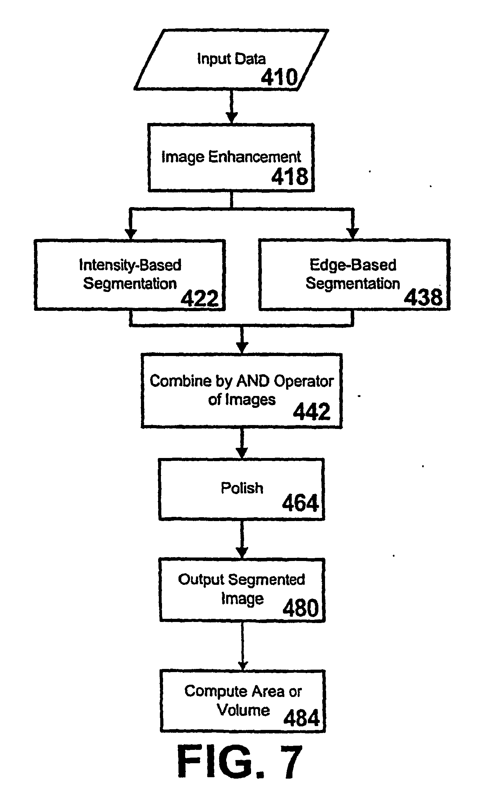

FIGURE 7 shows a block diagram overview the image enhancement, segmentation, and polishing algorithms of the amniotic fluid volume measuring system. The enhancement, segmentation, and polishing algorithms are applied to each scanplane 210 or to the entire scan cone 240 to automatically obtain amniotic fluid regions. For scanplanes substantially equivalent to scanplane 210, the algorithms are expressed in two-dimensional terms and use formulas to convert scanplane pixels (picture elements) into area units. For the scan cones substantially equivalent to the 3D conic array 240, the algorithms are expressed in three-dimensional terms and use formulas to convert voxels (volume elements) into volume units.

The algorithms expressed in 2D terms are used during the targeting phase where the operator trans-abdominally positions and repositions the transceiver 10 to obtain real-time feedback about the amniotic fluid area in each scanplane. The algorithms expressed in 3D terms are used to obtain the total amniotic fluid volume computed from the voxels contained within the calculated amniotic fluid regions in the 3D conic array 240.

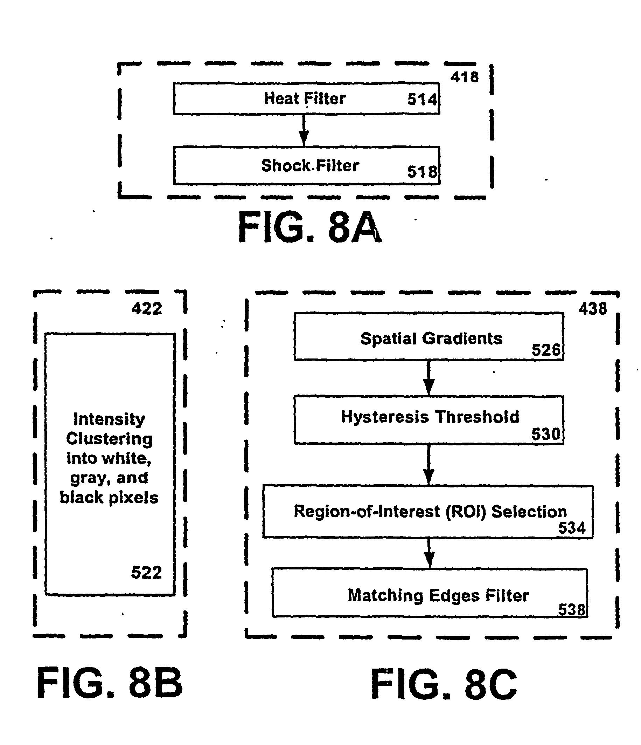

FIGURE 7 represents an overview of a preferred method of the invention and includes a sequence of algorithms, many of which have sub-algorithms described in more specific detail in FIGURES 8A-F. FIGURE 7 begins with inputting data of an unprocessed image at step 410. After unprocessed image data 410 is entered (e.g., read from memory, scanned, or otherwise acquired), it is automatically subjected to an image enhancement algorithm 418 that reduces the noise in the data (including speckle noise) using one or more equations while preserving the salient edges on the image using one or more additional equations. Next, the enhanced images are segmented by two different methods whose results are eventually combined. A first segmentation method applies an intensity-based segmentation algorithm 422 that determines all pixels that are potentially fluid pixels based on their intensities. A second segmentation method applies an edge-based segmentation algorithm 438 that relies on detecting the fluid and tissue interfaces. The images obtained by the first segmentation algorithm 422 and the images obtained by the second segmentation algorithm 438 are brought together via a combination algorithm 442 to provide a substantially segmented image. The segmented image obtained from the combination algorithm 442 are then subjected to a polishing algorithm 464 in which the segmented image is cleaned-up by filling gaps with pixels and removing unlikely regions. The image obtained from the polishing algorithm 464 is outputted 480 for calculation of areas and volumes of segmented regions-of-interest. Finally the area or the volume of the segmented region-of-interest is computed 484 by multiplying pixels by a first resolution factor to obtain area, or voxels by a second resolution factor to obtain volume. For example, for pixels having a size of 0.8mm by 0.8 mm, the first resolution or conversion factor for pixel area is equivalent to 0.64 mm$^2$, and the second resolution or conversion factor for voxel volume is equivalent to 0.512 mm$^3$. Different unit lengths for pixels and voxels may be assigned, with a proportional change in pixel area and voxel volume conversion factors.

The enhancement, segmentation and polishing algorithms depicted in FIGURE 7 for measuring amniotic fluid areas or volumes are not limited to scanplanes assembled into rotational arrays equivalent to the 3D array 240. As additional examples, the enhancement, segmentation and polishing algorithms depicted in FIGURE 7 apply to translation arrays and wedge arrays. Translation arrays are substantially rectilinear image plane slices from incrementally repositioned ultrasound transceivers that are configured to acquire ultrasound rectilinear scanplanes separated by regular or irregular rectilinear spaces. The translation arrays can be made from transceivers configured to advance incrementally, or may be hand-positioned incrementally by an operator. The operator obtains a wedge array from ultrasound transceivers configured to acquire wedge-shaped scanplanes separated by regular or irregular angular spaces, and either mechanistically advanced or hand-tilted incrementally. Any number of scanplanes can be either translationally assembled or wedge-assembled ranges, but preferably in ranges greater than 2 scanplanes.

Other preferred embodiments of the enhancement, segmentation and polishing algorithms depicted in FIGURE 7 may be applied to images formed by line arrays, either spiral distributed or reconstructed random-lines. The line arrays are defined using points identified by the coordinates expressed by the three parameters, P($r,\phi,\theta$), where the values or $r$, $\phi$, and $\theta$ can vary.

The enhancement, segmentation and polishing algorithms depicted in FIGURE 7 are not limited to ultrasound applications but may be employed in other imaging technologies utilizing scanplane arrays or individual scanplanes. For example, biological-based and non-biological-based images acquired using infrared, visible light, ultraviolet light, microwave, x-ray computed tomography, magnetic resonance, gamma rays, and positron emission are images suitable for the algorithms depicted in FIGURE 7. Furthermore, the algorithms depicted in FIGURE 7 can be applied to facsimile transmitted images and documents.

FIGURES 8A-E depict expanded details of the preferred embodiments of enhancement, segmentation, and polishing algorithms described in Figure 7. Each of the following greater detailed algorithms are either implemented on the transceiver 10 itself or are implemented on the host computer 52 or on the server 56 computer to which the ultrasound data is transferred.

FIGURE 8A depicts the sub-algorithms of Image Enhancement. The sub-algorithms include a heat filter 514 to reduce noise and a shock filter 518 to sharpen edges. A combination of the heat and shock filters works very well at reducing noise and sharpening the data while preserving the significant discontinuities. First, the noisy signal is filtered using a 1D heat filter (Equation E1 below), which results in the reduction of noise and smoothing of edges. This step is followed by a shock-filtering step 518 (Equation E2 below), which results in the sharpening of the blurred signal. Noise reduction and edge sharpening is achieved by application of the following equations E1-E2. The algorithm of the heat filter 514 uses a heat equation E1. The heat equation E1 in partial differential equation (PDE) form for image processing is expressed as:

$$\frac{\partial u}{\partial t} = \frac{\partial^2 u}{\partial x^2} + \frac{\partial^2 u}{\partial y^2}, \qquad\qquad \text{E 1}$$

where u is the image being processed. The image u is 2D, and is comprised of an array of pixels arranged in rows along the x-axis, and an array of pixels arranged in columns along the y-axis. The pixel intensity of each pixel in the image u has an initial input image pixel intensity (I) defined as $u_0 = I$. The value of $I$ depends on the application, and commonly occurs within ranges consistent with the application. For example, $I$ can be as low as 0 to 1, or occupy middle ranges between 0 to 127 or 0 to 512. Similarly, $I$ may have values occupying higher ranges of 0 to 1024 and 0 to 4096, or greater.

The heat equation E1 results in a smoothing of the image and is equivalent to the Gaussian filtering of the image. The larger the number of iterations that it is applied for the more the input image is smoothed or blurred and the more the noise that is reduced.

The shock filter 518 is a PDE used to sharpen images as detailed below. The two dimensional shock filter E2 is expressed as:

$$\frac{\partial u}{\partial t} = -F(\ell(u))\|\nabla u\|,$$ 
E 2

where u is the image processed whose initial value is the input image pixel intensity (I): $u_0 = I$ where the $\ell(u)$ term is the Laplacian of the image $u$, $F$ is a function of the Laplacian, and $\|\nabla u\|$ is the 2D gradient magnitude of image intensity defined by equation E3.

$$\|\nabla u\| = \sqrt{u_x^2 + u_y^2},$$ 
E3

where

$u^2_x$ = the square of the partial derivative of the pixel intensity (u) along the x-axis,
$u^2_y$ = the square of the partial derivative of the pixel intensity (u) along the y-axis,

the Laplacian $\ell(u)$ of the image, u, is expressed in equation E4 as

$$\ell(u) = u_{xx}u_x^2 + 2u_{xy}u_x u_y + u_{yy}u_y^2$$ 
E 4

where equation E4 relates to equation E1 as follows:

$u_x$ is the first partial derivative $\dfrac{\partial u}{\partial x}$ of u along the x-axis,

$u_y$ is the first partial derivative $\dfrac{\partial u}{\partial y}$ of u along the y-axis,

$u_x{}^2$ is the square of the first partial derivative $\dfrac{\partial u}{\partial x}$ of u along the x-axis,

$u_y{}^2$ is the square of the first partial derivative $\dfrac{\partial u}{\partial y}$ of u along the y-axis,

$u_{xx}$ is the second partial derivative $\dfrac{\partial^2 u}{\partial x^2}$ of u along the x-axis,

$u_{yy}$ is the second partial derivative $\dfrac{\partial^2 u}{\partial y^2}$ of u along the y-axis,

$u_{xy}$ is cross multiple first partial derivative $\dfrac{\partial u}{\partial x dy}$ of u along the x and y axes, and

the sign of the function $F$ modifies the Laplacian by the image gradient values selected to avoid placing spurious edges at points with small gradient values:

$$F(\ell(u)) = 1, \text{ if } \ell(u) > 0 \text{ and } \|\nabla u\| > t$$

$$= \text{-}1, \text{ if } \ell(u) < 0 \text{ and } \|\nabla u\| > t$$

$$= 0, \text{ otherwise}$$

where $t$ is a threshold on the pixel gradient value $\|\nabla u\|$.

The combination of heat filtering and shock filtering produces an enhanced image ready to undergo the intensity-based and edge-based segmentation algorithms as discussed below.

FIGURE 8B depicts the sub-algorithms of Intensity-Based Segmentation (step 422 in FIGURE 7). The intensity-based segmentation step 422 uses a "k-means" intensity clustering 522 technique where the enhanced image is subjected to a categorizing "k-means" clustering algorithm. The "k-means" algorithm categorizes pixel intensities into white, gray, and black pixel groups. Given the number of desired clusters or groups of intensities (k), the k-means algorithm is an iterative algorithm comprising four steps:

1. Initially determine or categorize cluster boundaries by defining a minimum and a maximum pixel intensity value for every white, gray, or black pixels into groups or k-clusters that are equally spaced in the entire intensity range.

2. Assign each pixel to one of the white, gray or black k-clusters based on the currently set cluster boundaries.

3. Calculate a mean intensity for each pixel intensity k-cluster or group based on the current assignment of pixels into the different k-clusters. The calculated mean intensity is defined as a cluster center. Thereafter, new cluster boundaries are determined as mid points between cluster centers.

4. Determine if the cluster boundaries significantly change locations from their previous values. Should the cluster boundaries change significantly from their previous values, iterate back to step 2, until the cluster centers do not change significantly between iterations. Visually, the clustering process is manifest by the segmented image and repeated iterations continue until the segmented image does not change between the iterations.

The pixels in the cluster having the lowest intensity value - the darkest cluster - are defined as pixels associated with amniotic fluid. For the 2D algorithm, each image is clustered independently of the neighboring images. For the 3D algorithm, the entire volume is clustered together. To make this step faster, pixels are sampled at 2 or any multiple sampling rate factors before determining the cluster boundaries. The cluster boundaries determined from the down-sampled data are then applied to the entire data.

FIGURE 8C depicts the sub-algorithms of Edge-Based Segmentation (step 438 in FIGURE 7) and uses a sequence of four sub-algorithms. The sequence includes a spatial gradients 526 algorithm, a hysteresis threshold 530 algorithm, a Region-of-Interest (ROI) 534 algorithm, and a matching edges filter 538 algorithm.

The spatial gradient 526 computes the x-directional and y-directional spatial gradients of the enhanced image. The Hysteresis threshold 530 algorithm detects salient edges. Once the edges are detected, the regions defined by the edges are selected by a user employing the ROI 534 algorithm to select regions-of-interest deemed relevant for analysis.

Since the enhanced image has very sharp transitions, the edge points can be easily determined by taking x- and y- derivatives using backward differences along x- and y-directions. The pixel gradient magnitude $\|\nabla I\|$ is then computed from the x- and y-derivative image in equation E5 as:

$$\|\nabla I\| = \sqrt{I_x^2 + I_y^2} \qquad\qquad \text{E5}$$

Where $I_x^2$ = the square of x-derivative of intensity; and $I_y^2$ = the square of y-derivative of intensity along the y-axis.

Significant edge points are then determined by thresholding the gradient magnitudes using a hysteresis thresholding operation. Other thresholding methods could also be used. In hysteresis thresholding 530, two threshold values, a lower threshold and a higher threshold, are used. First, the image is thresholded at the lower threshold value and a connected component labeling is carried out on the resulting image. Next, each connected edge component is preserved which has at least one edge pixel having a gradient magnitude greater than the upper threshold. This kind of thresholding scheme is good at retaining long connected edges that have one or more high gradient points.

In the preferred embodiment, the two thresholds are automatically estimated. The upper gradient threshold is estimated at a value such that at most 97% of the image pixels are marked as non-edges. The lower threshold is set at 50% of the value of the upper threshold. These percentages could be different in different implementations. Next, edge points that lie within a desired region-of-interest are selected 534. This region of interest selection 534 excludes points lying at the image boundaries and points lying too close to or too far from the transceiver 10. Finally, the matching edge filter 538 is applied to remove outlier edge points and fill in the area between the matching edge points.

The edge-matching algorithm 538 is applied to establish valid boundary edges and remove spurious edges while filling the regions between boundary edges. Edge points on an image have a directional component indicating the direction of the gradient. Pixels in scanlines crossing a boundary edge location will exhibit two gradient transitions depending on the pixel intensity directionality. Each gradient transition is given a positive or negative value depending on the pixel intensity directionality. For example, if the scanline approaches an echo reflective bright wall from a darker region, then an ascending transition is established as the pixel intensity gradient increases to a maximum value, i.e., as the transition ascends from a dark region to a bright region. The ascending transition is given a positive numerical value. Similarly, as the scanline recedes from the echo reflective wall, a descending transition is established as the pixel intensity gradient decreases to or approaches a minimum value. The descending transition is given a negative numerical value.

Valid boundary edges are those that exhibit ascending and descending pixel intensity gradients, or equivalently, exhibit paired or matched positive and negative numerical values. The valid boundary edges are retained in the image. Spurious or invalid boundary edges do not exhibit paired ascending-descending pixel intensity gradients, i.e., do not exhibit paired or matched positive and negative numerical values. The spurious boundary edges are removed from the image.

For amniotic fluid volume related applications, most edge points for amniotic fluid surround a dark, closed region, with directions pointing inwards towards the center of the region. Thus, for a convex-shaped region, the direction of a gradient for any edge point, the edge point having a gradient direction approximately opposite to the current point represents the matching edge point. Those edge points exhibiting an assigned positive and negative value are kept as valid edge points on the image because the negative value is paired with its positive value counterpart. Similarly, those edge point candidates having unmatched values, i.e., those edge point candidates not having a negative-positive value pair, are deemed not to be true or valid edge points and are discarded from the image.

The matching edge point algorithm 538 delineates edge points not lying on the boundary for removal from the desired dark regions. Thereafter, the region between any two matching edge points is filled in with non-zero pixels to establish edge-based segmentation. In a preferred embodiment of the invention, only edge points whose directions are primarily oriented co-linearly with the scanline are sought to permit the detection of matching front wall and back wall pairs.

Returning to FIGURE 7, once Intensity-Based 422 and Edge-Based Segmentation 438 is completed, both segmentation methods use a combining step that combines the results of intensity-based segmentation 422 step and the edge-based segmentation 438 step using an AND Operator of Images 442. The AND Operator of Images 442 is achieved by a pixel-wise Boolean AND operator 442 step to produce a segmented image by computing the pixel intersection of two images. The Boolean AND operation 442 represents the pixels as binary numbers and the corresponding assignment of an assigned intersection value as a binary number 1 or 0 by the combination of any two pixels. For example, consider any two pixels, say $pixel_A$ and $pixel_B$, which can have a 1 or 0 as assigned values. If $pixel_A$'s value is 1, and $pixel_B$'s value is 1, the assigned intersection value of $pixel_A$ and $pixel_B$ is 1. If the binary value of $pixel_A$ and $pixel_B$ are both 0, or if either pixels or $pixel_B$ is 0, then the assigned intersection value of $pixel_A$ and $pixel_B$ is 0. The Boolean AND operation 542 takes the binary any two digital images as input, and outputs a third image with the pixel values made equivalent to the intersection of the two input images.

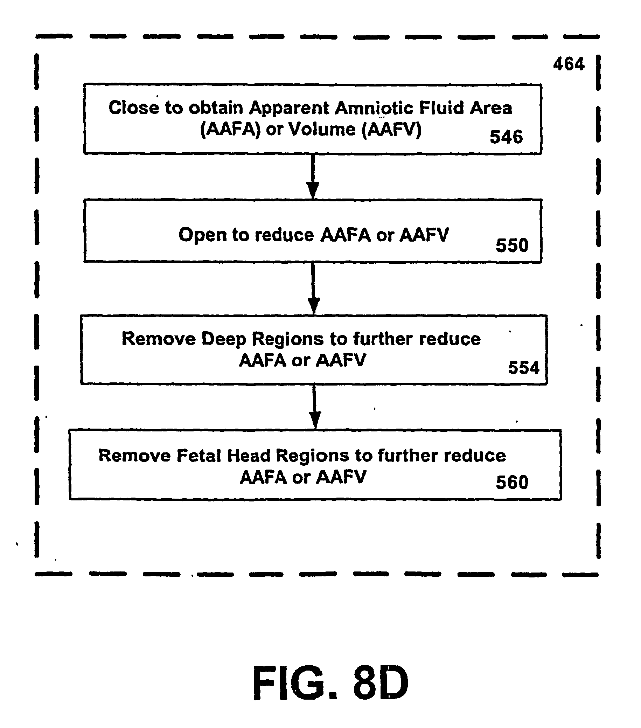

Upon completion of the AND Operator of Images 442 algorithm, the polish 464 algorithm of FIGURE 7 is comprised of multiple sub-algorithms. FIGURE 8D depicts the sub-algorithms of the Polish 464 algorithm, including a Close 546 algorithm, an Open 550 algorithm, a Remove Deep Regions 554 algorithm, and a Remove Fetal Head Regions 560 algorithm.

Closing and opening algorithms are operations that process images based on the knowledge of the shape of objects contained on a black and white image, where white represents foreground regions and black represents background regions. Closing serves to remove background features on the image that are smaller than a specified size. Opening serves to remove foreground features on the image that are smaller than a specified size. The size of the features to be removed is specified as an input to these operations. The opening algorithm 550 removes unlikely amniotic fluid regions from the segmented image based on *a-priori* knowledge of the size and location of amniotic fluid pockets.

Referring to FIGURE 8D, the closing 546 algorithm obtains the Apparent Amniotic Fluid Area (AAFA) or Volume (AAFV) values. The AAFA and AAFV values are "Apparent" and maximal because these values may contain region areas or region volumes of non-amniotic origin unknowingly contributing to and obscuring what otherwise would be the true amniotic fluid volume. For example, the AAFA and AAFV values contain the true amniotic volumes, and possibly as well areas or volumes due to deep tissues and undetected fetal head volumes. Thus the apparent area and volume values require correction or adjustments due to unknown contributions of deep tissue and of the fetal head in order to determine an Adjusted Amniotic Fluid Area (AdAFA) value or Volume (AdAVA) value 568.

The AdAFA and AdAVA values obtained by the Close 546 algorithm are reduced by the morphological opening algorithm 550. Thereafter, the AdAFA and AdAVA values are further reduced by removing areas and volumes attributable to deep regions by using the Remove Deep Regions 554 algorithm. Thereafter, the polishing algorithm 464 continues by applying a fetal head region detection algorithm 560.

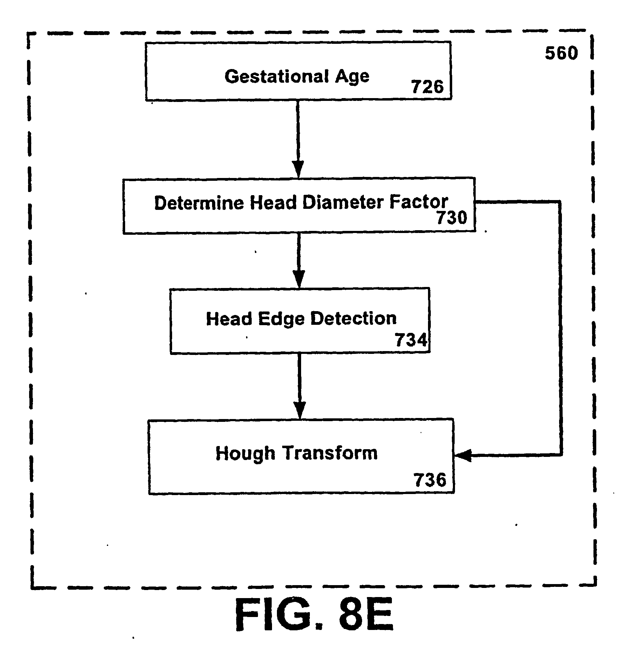

FIGURE 8E depicts the sub-algorithms of the Remove Fetal Head Regions sub-algorithm 560. The basic idea of the sub-algorithms of the fetal head detection algorithm 560 is that the edge points that potentially represent a fetal skull are detected. Thereafter, a circle finding algorithm to determine the best-fitting circle to these fetal skull edges is implemented. The radii of the circles that are searched are known a priori based on the fetus' gestational age. The best fitting circle whose fitting metric lies above a certain prespecified threshold is marked as the fetal head and the region inside this circle is the fetal head region. The algorithms include a gestational Age 726 input, a determine head diameter factor 730 algorithm, a Head Edge Detection algorithm, 734, and a Hough transform procedure 736.

Fetal brain tissue has substantially similar ultrasound echo qualities as presented by amniotic fluid. If not detected and subtracted from amniotic fluid volumes, fetal brain tissue volumes will be measured as part of the total amniotic fluid volumes and lead to an overestimation and false diagnosis of oligo or poly-hyraminotic conditions. Thus detecting fetal head position, measuring fetal brain matter volumes, and deducting the fetal brain matter volumes from the amniotic fluid volumes to obtain a corrected amniotic fluid volume serves to establish accurately measure amniotic fluid volumes.

The gestational age input 726 begins the fetal head detection algorithm 560 and uses a head dimension table to obtain ranges of head bi-parietal diameters (BPD) to search for (e.g., 30 week gestational age corresponds to a 6 cm head diameter). The head diameter range is input to both the Head Edge Detection, 734, and the Hough Transform, 736., The head edge detection 734 algorithm seeks out the distinctively bright ultrasound echoes from the anterior and posterior walls of the fetal skull while the Hough Transform algorithm, 736, finds the fetal head using circular shapes as models for the fetal head in the Cartesian image (pre-scan conversion to polar form).

Scanplanes processed by steps 522, 538, 530, are input to the head edge detection step 734. Applied as the first step in the fetal head detection algorithm 734 is the detection of the potential head edges from among the edges found by the matching edge filter. The matching edge 538 filter outputs pairs of edge points potentially belonging to front walls or back walls. Not all of these walls correspond to fetal head locations. The edge points representing the fetal head are determined using the following heuristics:

(1) Looking along a one dimensional A-mode scan line, fetal head locations present a corresponding matching gradient in the opposing direction within a short distance approximately the same size as the thickness of the fetal skull. This distance is currently set to a value 1 cm.
(2) The front wall and the back wall locations of the fetal head are within a range of diameters corresponding to the expected diameter 730 for the gestational age 726 of the fetus. Walls that are too close or too far are not likely to be head locations.
(3) A majority of the pixels between the front and back wall locations of the fetal head lie within the minimum intensity cluster as defined by the output of the clustering algorithm 422. The percentage of pixels that need to be dark is currently defined to be 80%.

The pixels found satisfying these features are then vertically dilated to produce a set of thick fetal head edges as the output of Head Edge Detection, 734.

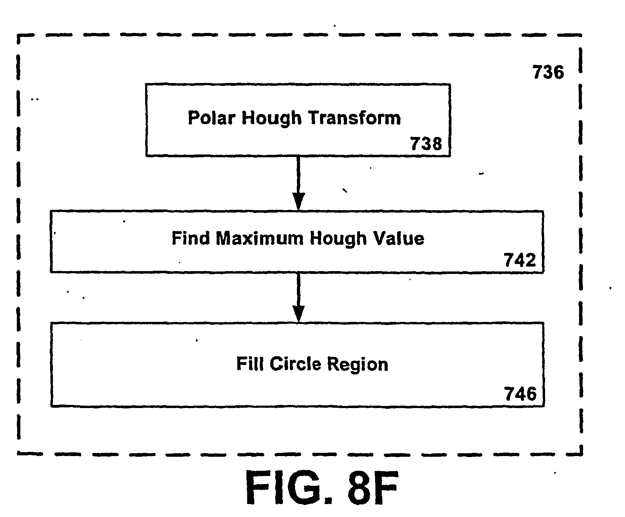

FIGURE 8F depicts the sub-algorithms of the Hough transform procedure 736. The sub-algorithms include a Polar Hough Transform 738 algorithm, a find maximum Hough value 742 algorithm 742, and a fill circle region 746. The Polar Hough Transform algorithm looks for fetal head structures in polar coordinate terms by converting from Cartesian coordinates using a plurality of equations. The fetal head, which appears like a circle in a 3D scan-converted Cartesian coordinate image, has a different shape in the pre-scan converted polar space. The fetal head shape is expressed in terms of polar coordinate terms explained as follows:

The coordinates of a circle in the Cartesian space (x,y) with center $(x_0, y_0)$ and radius R are defined for an angle θ are derived and defined in equation E5 as:

$$x = R\cos\theta + x_0$$
$$y = R\sin\theta + y_0$$
$$\Rightarrow (x - x_0)^2 + (y - y_0)^2 = R^2 \qquad\qquad E5$$

In polar space, the coordinates $(r, \phi)$, with respect to the center $(r_0, \phi_0)$, are derived and defined in equation E6 as:

$$r\sin\phi = R\cos\theta + r_0\sin\phi_0$$
$$r\cos\phi = R\sin\theta + r_0\cos\phi_0$$
$$\Rightarrow (r\sin\phi - r_0\sin\phi_0)^2 + (r\cos\phi - r_0\cos\phi_0)^2 = R^2 \qquad\qquad E6$$

The Hough transform 736 algorithm using equations E5 and E6 attempts to find the best-fit circle to the edges of an image. A circle in the polar space is defined by a set of three parameters, $(r_0, \phi_0, R)$ representing the center and the radius of the circle.

The basic idea for the Hough transform 736 is as follows. Suppose a circle is sought having a fixed radius (say, R1) for which the best center of the circle is similarly sought. Now, every edge point on the input image lies on a potential circle whose center lays R1 pixels away from it. The set of potential centers themselves form a circle of radius R1 around each edge pixel. Now, drawing potential circles of radius R1 around each edge pixel, the point at which most circles intersect, a center of the circle that represents a best-fit circle to the given edge points is obtained. Therefore, each pixel in the Hough transform output contains a likelihood value that is simply the count of the number of circles passing through that point.

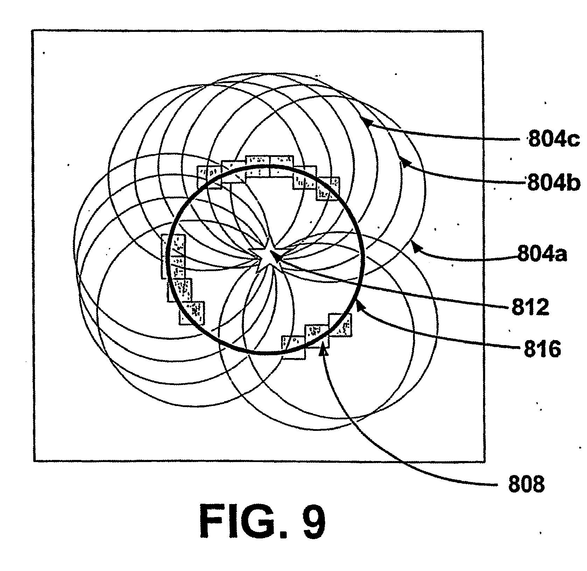

FIGURE 9 illustrates the Hough Transform 736 algorithm for a plurality of circles with a fixed radius in a Cartesian coordinate system. A portion of the plurality of circles is represented by a first circle 804a, a second circle 804b, and a third circle 804c. A plurality of edge pixels are represented as gray squares and an edge pixel 808 is shown. A circle is drawn around each edge pixel to distinguish a center location 812 of a best-fit circle 816 passing through each edge pixel point; the point of the center location through which most such circles pass (shown by a gray star 812) is the center of the best-fit circle 816 presented as a thick dark line. The circumference of the best fit circle 816 passes substantially through is central portion of each edge pixel, represented as a-series of squares substantially equivalent to the edge pixel 808.

This search for best fitting circles can be easily extended to circles with varying radii by adding one more degree of freedom - however, a discrete set of radii around the mean radii for a given gestational age makes the search significantly faster, as it is not necessary to search all possible radii.

The next step in the head detection algorithm is selecting or rejecting best-fit circles based on its likelihood, in the find maximum Hough Value 742 algorithm. The greater the number of circles passing through a given point in the Hough-space, the more likely it is to be the center of a best-fit circle. A 2D metric as a maximum Hough value 742 of the Hough transform 736 output is defined for every image in a dataset. The 3D metric is defined as the maximum of the 2D metrics for the entire 3D dataset. A fetal head is selected on an image depending on whether its 3D metric value exceeds a preset 3D threshold and also whether the 2D metric exceeds a preset 2D threshold. The 3D threshold is currently set at 7 and the 2D threshold is currently set at 5. These thresholds have been determined by extensive training on images where the fetal head was known to be present or absent.

Thereafter, the fetal head detection algorithm concludes with a fill circle region 746 that incorporates pixels to the image within the detected circle. The fill circle region 746 algorithm fills the inside of the best fitting polar circle. Accordingly, the fill circle region 746 algorithm encloses and defines the area of the fetal brain tissue, permitting the area and volume to be calculated and deducted via algorithm 554 from the apparent amniotic fluid area and volume (AAFA or AAFV) to obtain a computation of the corrected amniotic fluid area or volume via algorithm 484.

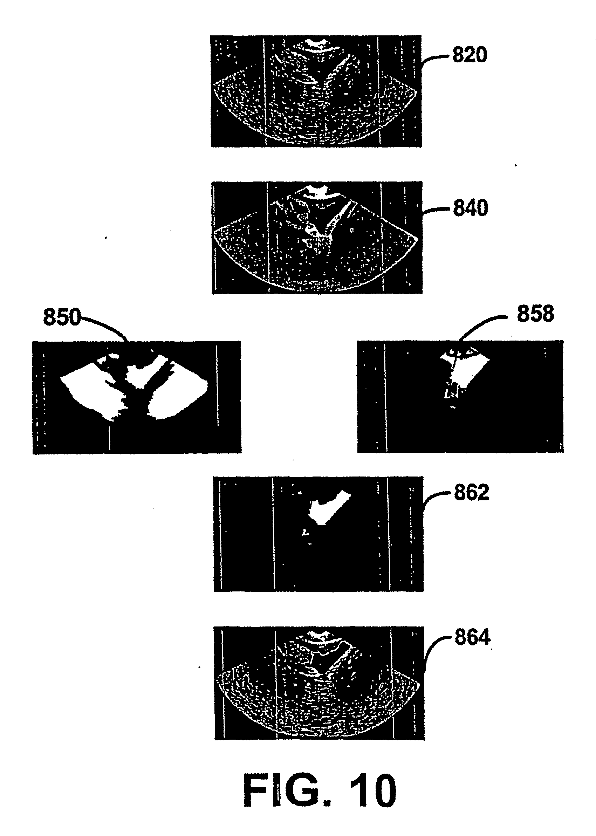

FIGURE 10 shows the results of sequentially applying the algorithm steps of FIGURES 7 and 8A-D on an unprocessed sample image 820 presented within the confines of a scanplane substantially equivalent to the scanplane 210. The results of applying the heat filter 514 and shock filter 518 in enhancing the unprocessed sample is shown in enhanced image 840. The result of intensity-based segmentation algorithms 522 is shown in image 850. The results of edge-based segmentation 438 algorithm using sub-algorithms 526, 530, 534 and 538 of the enhanced image 840 is shown in segmented image 858. The result of the combination 442 utilizing the Boolean AND images 442 algorithm is shown in image 862 where white represents the amniotic fluid area. The result of applying the polishing 464 algorithm employing algorithms 542, 546, 550, 554, 560, and 564 is shown in image 864, which depicts the amniotic fluid area overlaid on the unprocessed sample image 810.

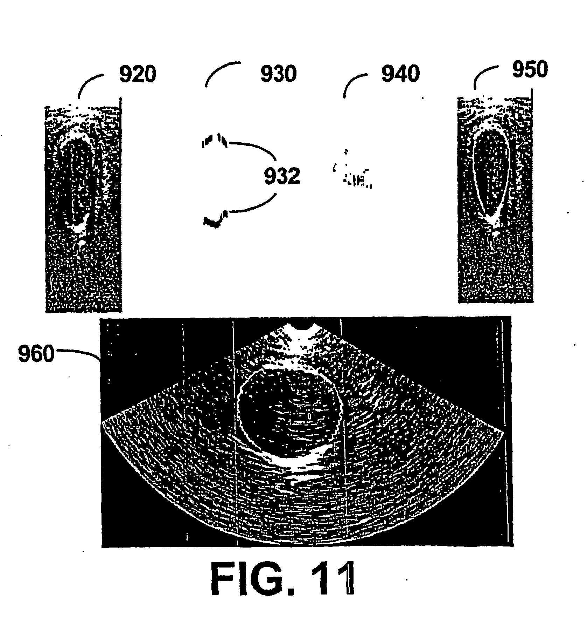

FIGURE 11 depicts a series of images showing the results of the above method to automatically detect, locate, and measure the area and volume of a fetal head using the algorithms outlined in FIGURES 7 and 8A-F. Beginning with an input image in polar coordinate form 920, the fetal head image is marked by distinctive bright echoes from the anterior and posterior walls of the fetal skull and a circular shape of the fetal head in the Cartesian image. The fetal head detection algorithm 734 operates on the polar coordinate data (i.e., pre-scan version, not yet converted to Cartesian coordinates).

An example output of applying the head edge detection 734 algorithm to detect potential head edges is shown in image 930. Occupying the space between the anterior and posterior walls are dilated black pixels 932 (stacks or short lines of black pixels representing thick edges). An example of the polar Hough transform 738 for one actual data sample for a specific radius is shown in polar coordinate image 940.

An example of the best-fit circle on real data polar data is shown in polar coordinate image 950 that has undergone the find maximum Hough value step 742. The polar coordinate image 950 is scan-converted to a Cartesian data in image 960 where the effects of finding maximum Hough value 742 algorithm are seen in Cartesian format.

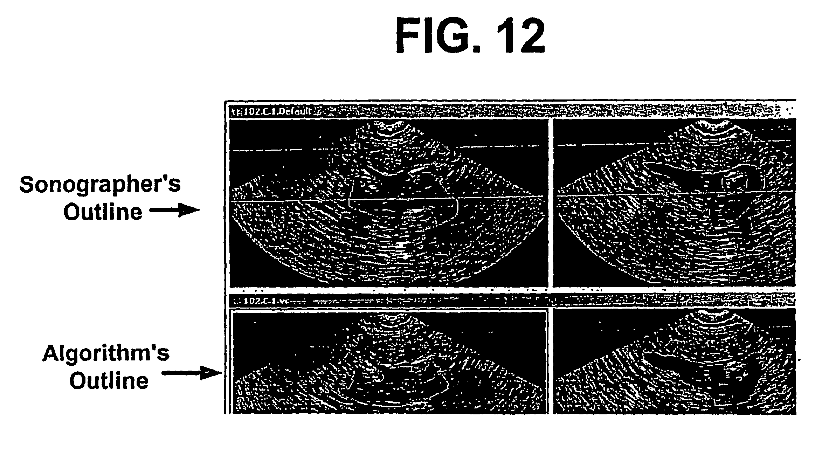

FIGURE 12 presents a 4-panel series of sonographer amniotic fluid pocket outlines compared to the algorithm's output in a scanplane equivalent to scanplane 210. The top two panels depict the sonographer's outlines of amniotic fluid pockets obtained by manual interactions with the display while the bottom two panels show the resulting amniotic fluid boundaries obtained from the instant invention's automatic application of 2D algorithms, 3D algorithms, combination heat and shock filter algorithms, and segmentation algorithms.

After the contours on all the images have been delineated, the volume of the segmented structure is computed. Two specific techniques for doing so are disclosed in detail in U.S. Pat. No. 5,235,985 to McMorrow et al, herein incorporated by reference. This patent provides detailed explanations for non-invasively transmitting, receiving and processing ultrasound for calculating volumes of anatomical structures.

Demonstrations of the algorithmic manipulation of pixels of the present invention are provided in *Appendix 1: Examples of Algorithmic Steps.* Source code of the algorithms of the present invention is provided in *Appendix 2: Matlab Source Code.*

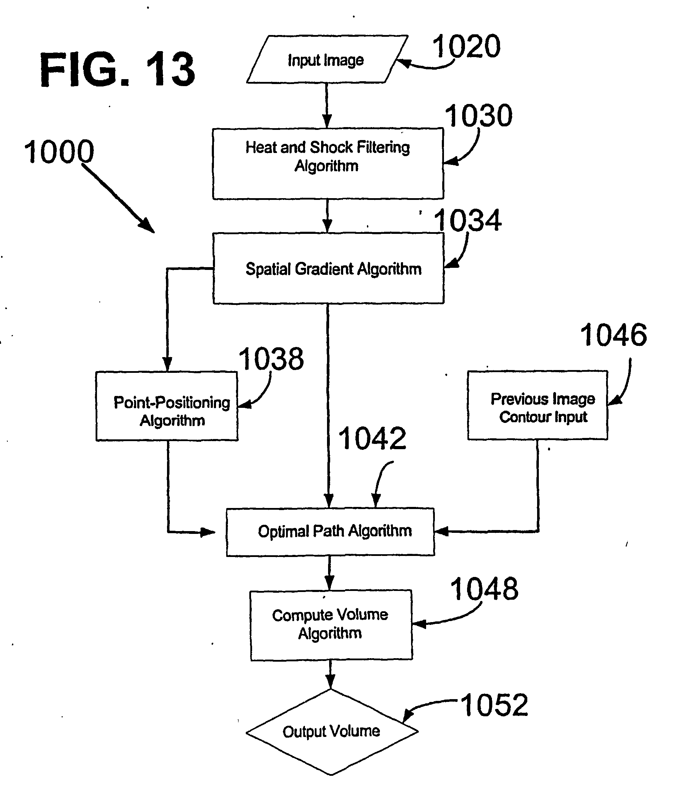

Figure 13 shows an overview of a segmentation algorithm 1000. The segmentation algorithm 1000 begins with the receiving of an input image 1020, to which a heat and shock filtering algorithm 1030 is applied to the input image 1020. Thereafter, a spatial gradient algorithm 1034 is applied to the image that has been heat and shock filtered. At this juncture, initial edge points are located in a point-positioning algorithm 1038 to the boundary of the organ structure within the image. Thereafter an optimal path algorithm 1042 is applied to determine the best path to connect the initial edge points assigned by the point-positioning algorithm 1038. This optimal path algorithm 1042 also uses the result of the spatial gradient algorithm 1034 as an input. For images already having a drawn contour, a previous image contour input 1046 is inputted to the optimal path algorithm 1042. Thereafter, a compute volume algorithm 1048 is applied to the image, and the computed volume is reported as an output volume 1052.

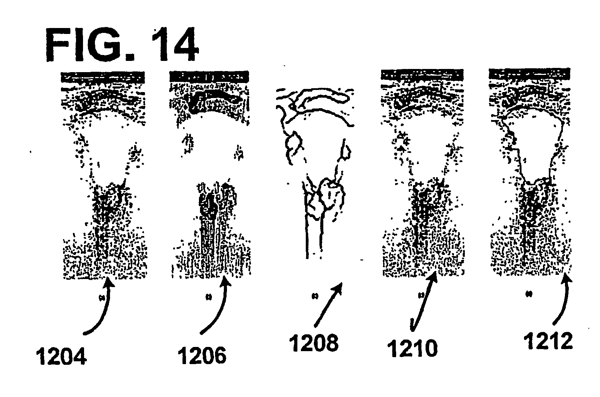

Figure 14 shows the segmentation algorithm applied to a sample image. An original bladder image 1204 acquires more contrast as shown in a bladder image 1206 once subjected to the heat and shock filtering algorithms. Thereafter, spatial gradients are depicted as shown in a bladder image 1208. Initial points are placed as shown in a bladder image 1210 when the point-positioning algorithm is applied. The point-positioning algorithm includes the radial method. Finally, a contour along the boundary where the points have been connected by applying the optimal path algorithm is shown in a bladder image 1212.

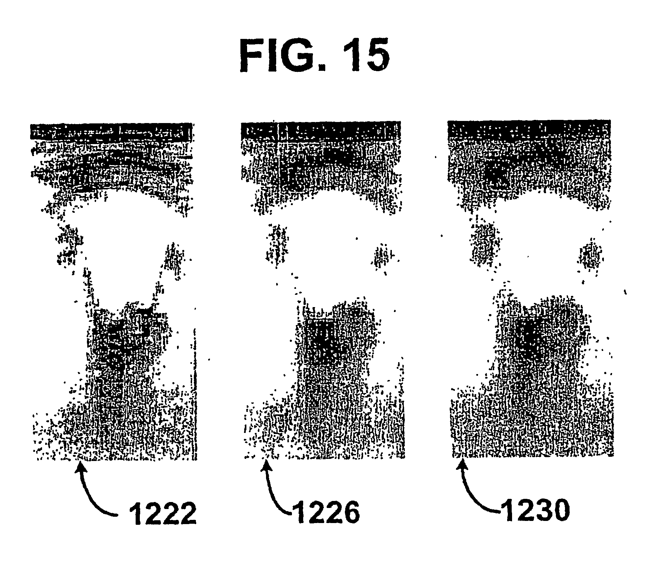

The effects of applying the heat filtering algorithm is shown in Figure 15. The effect of 10 iterations of heat algorithm is shown in image 1222. The effect of 50 iterations is shown in image 1226. The effect of 100 iterations is shown in image 1230. With more iterations of heat filtering algorithms, the more the image becomes blurry.

For scanplanes substantially equivalent to scanplane 210, the algorithms are expressed in two-dimensional terms and use formulas to convert scanplane pixels (picture elements) into area units. For the scan cones substantially equivalent to the 3D conic array 240, the algorithms are expressed in three-dimensional terms and use formulas to convert voxels (volume elements) into volume units. The heat-filtering algorithm is determined one of the simplest PDE for image processing is the heat equation as shown in equation E7:

$$\frac{\partial u}{\partial t} = \frac{\partial^2 u}{\partial x^2} + \frac{\partial^2 u}{\partial y^2}, \qquad\qquad \mathrm{E\ 7}$$

where $u$ is the image being processed whose initial value is the input image: $u_0 = I$.

The above PDE can be discretized using finite differences to give the following iterative scheme of equation E8:

$$\frac{u_{i,j}^{t+1} - u_{i,j}^{t}}{k} = (u_{i+1,j}^{t} - 2u_{i,j}^{t} + u_{i-1,j}^{t}) + (u_{i,j+1}^{t} - 2u_{i,j}^{t} + u_{i,j-1}^{t}) \qquad \text{E 8}$$

which can be simplified as shown in equation E9:

$$u_{i,j}^{t+1} = u_{i,j}^{t} + k(u_{i+1,j}^{t} + u_{i-1,j}^{t} + u_{i,j+1}^{t} + u_{i,j-1}^{t} - 4u_{i,j}^{t}) \qquad \text{E 9}$$

where k is the time step, and $u_{i,j}^{t}$ is the value of the pixel at coordinates (i,j) at time point (iteration) t.

This heat equation results in a smoothing of the image substantially equivalent to the Gaussian filtering of the image. The larger the number of iterations the more the input image is smoothed or blurred.

Shock filters are another type of PDE-based filters that were originally proposed by Osher and Rudin (Osher and Rudin 1990) for sharpening of signals and images. A one-dimensional shock filter is given as equation E10:

$$\frac{\partial u}{\partial t} = -sign(\frac{\partial^2 u}{dx^2})\left|\frac{\partial u}{dx}\right| \qquad \text{E 10}$$

where u is the image being processed, $\frac{\partial^2 u}{\partial x^2}$ is the second partial derivative of u, $\left|\frac{\partial u}{dx}\right|$ is the absolute value of the first derivative of u along the x-axis. Here, the signal is modified based on the sign of its second derivative and the magnitude of its first derivative. This filter cannot be discretized using the simple centered finite-difference scheme.

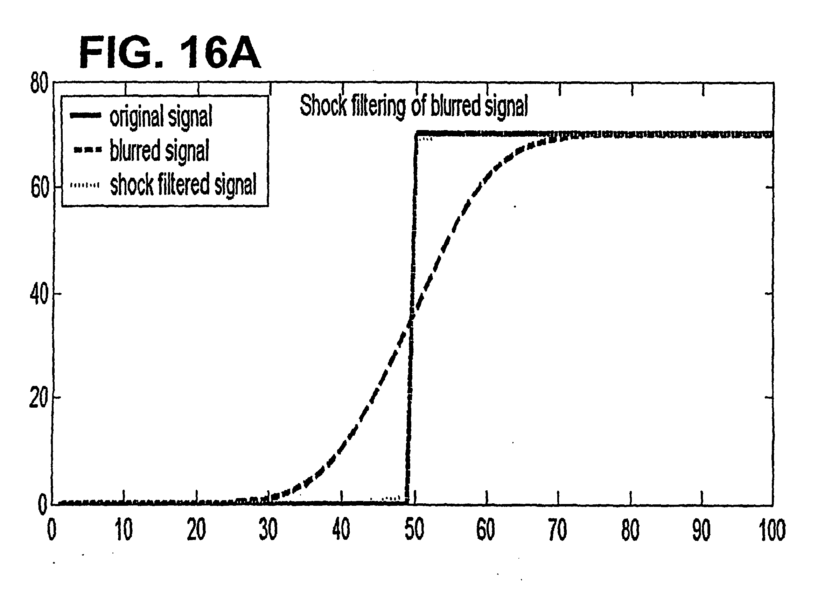

The result of applying this filter on noiseless blurred data is shown in Figure 16A. The x-axis shows the index coordinates of the signals while the y-axis shows the magnitude of the signals. The solid line is the original noiseless signal which was blurred with a Gaussian filter algorithm. The blurred signal is shown as the dotted line. A shock filter was then applied to the blurred signal and that results in the signal shown in light shaded line - it is virtually the same as the original signal and the edge is restored from the blurred signal.

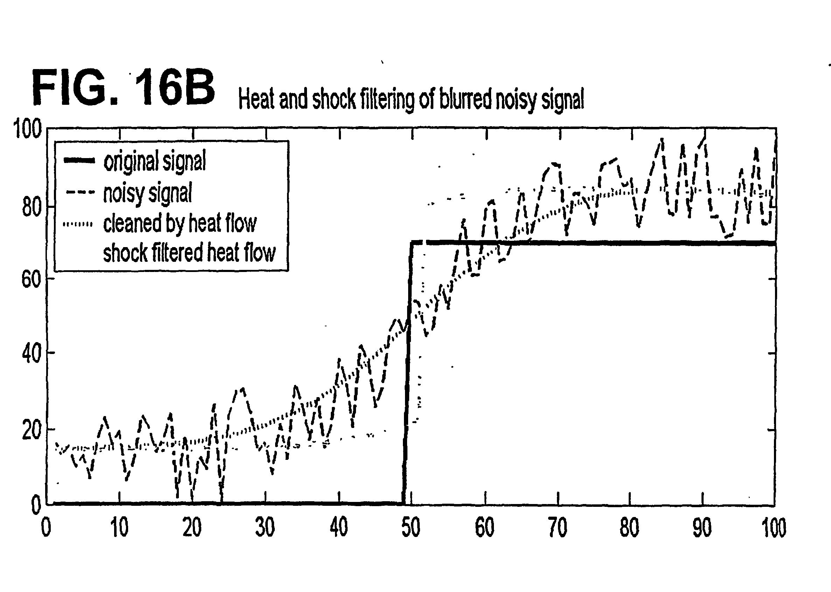

Applying this filter directly to noisy data, however, does not work and leads to many possible false edges. The combination of heat and shock filters would works very well at reducing noise and sharpening of a signal while preserving the significant discontinuities on a signal. First, the noisy signal is filtered using the 1D heat filter (Equation E1), which results in the reduction of noise and smoothing of edges. This step is followed by a shock-filtering step (Equation E4), which results in the sharpening of the blurred signal. A 1D simulated example of this combination heat/shock filter is shown in Figure 16B. The solid line is the original noiseless signal while the dotted dark line is the noisy blurred signal. The result of heat filtering the noisy signal by the gray dashed line. Finally, the result of shock filtering after heat filtering is shown by the gray dot-dashed line. Note that on the last signal, the significant edge has been preserved while the noise has been removed.

In two dimensions, the shock filter PDE specified by Osher and Rudin is:

$$\frac{\partial u}{\partial t} = -F(\ell(u))\|\nabla u\|, \qquad \text{E 11}$$

where $\ell(u)$ is the Laplacian of the image (u, F.) is a function of the Laplacian, and $\|\nabla u\|$ is the gradient magnitude. For the Laplacian, the second derivative in the direction of the gradient is used:

$$\ell(u) = u_{xx}u_x^2 + 2u_{xy}u_xu_y + u_{yy}u_y^2 \qquad \text{E 12}$$

For the function F, to avoid placing spurious edges at points with small gradient values as would occur by choosing the sign by selecting the sign of the Laplacian in the 1-D case, the following function is used instead:

$$F(\ell(u)) = 1, \text{ if } \ell(u) > 0 \text{ and} \|\nabla u\| > t$$

$$= -1, \text{ if } \ell(u) < 0 \text{ and} \|\nabla u\| > t$$

$$= 0, \text{ otherwise}$$

where t is a threshold on gradient values.

Once again, as in the 1D case, a combination of the heat and shock filters works very well at reducing noise and sharpening of a signal while preserving the significant edges on an image. Figure 14-image 1208 shows the results of applying the heat and shock filter on real ultrasound data.

The point-positioning algorithm uses two different methods for locating the initial edge points. One method looks for edges along radial lines and the other looks for edges along A-mode lines. The first method requires the user to specify a center point from which radial lines are drawn for an image, and the second method is completely automatic.

In the first method, to locate the initial edge points on an image, the image is radially sampled from the user marked center location. The radial lines are drawn at substantially in 30-degree increments. On each radial line, edge points are found by using the values obtained from the spatial gradient algorithm. A first point on each radial line whose gradient magnitude exceeds a pre-set threshold and which represents a transition from a dark area to a bright area. If no such point is found, that radial line is skipped. As shown in Figure 14 image 1210, the points are assigned to the edge locations.

In the second method, the A-mode lines starting from the transducer position are moved outwards. One-dimensional gradients and pixel intensity values $I$ are used along these A-mode lines to determine fluid wall locations. A hysteresis threshold is applied to the gradient values to determine the leading wall and the trailing wall of the structure.

Given two points on an image, p1 and p2, an optimal path finding algorithm finds the "best" path between the two points where "best" is the one that has the least cost. The cost function for a path can be defined to depend upon many factors such as, how close the path passes through edges on an image, the direction of the path, how smooth the path is, and what the length of the path is. A dynamic programming algorithm can be then used to locate the least cost path between p1 and p2.

The total cost function to be a weighted is defined by a sum of 3 terms as shown in equation E13:

$$C(p) = \alpha C_e(p) + \beta C_d(p) + \gamma C_c(p) \qquad\qquad \text{E 13}$$

where $\alpha, \beta$, *and* $\gamma$ are numerical values and the three cost terms are:

(1) Edge Distance Cost $C_e(p)$: This cost is defined such that paths closer to high gradient points, i.e., edges, are favored over other paths.

$$C_e(p) = \sum_{i=p1}^{p2} \left( \frac{1}{\mu + \|\nabla I_i\|} \right), \qquad\qquad \text{E 14}$$

where $I_i$ is the pixel intensity at point i, $\|\nabla I_i\|$ is the gradient magnitude of point i, and $\mu$ is a small constant. Since this term is the sum of inverse image gradients for each pixel on the path - this cost term implicitly favors shorter paths over longer paths.

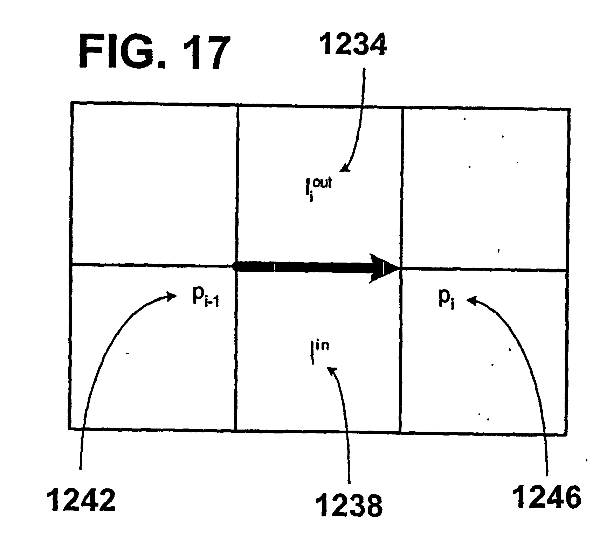

(2) Path Direction Cost: Since the path between $p^1$ and $p^2$ is a directed graph, a cost can be defined based on the orientation of the graph. The cost is defined to favor paths for which pixels on the inside of the path are dark and the pixels on the outside are bright (See Figure 17). The PDE formula for calculating the path direction cost is provided in equation E15.

$$C_d(p) = \sum_{i=p1}^{p2}(I_i^{in} - I_i^{out}),\qquad\text{E 15}$$

where $I_i^{in}$ and $I_i^{out}$ are the image intensities on the inside and the outside of the path connecting point $i$ and $i_{-1}$ as shown in Figure 17. Figure 17 depicts, at the pixel level, how the optimal path algorithm is applied. Direction of path proceeds from point 1242 ($p_{i-1}$) to point 1246 ($p_i$) between the outside pixel 1234 $(I_i^{out})$ and the inside pixel 1238 $(I_i^{in})$. Given two points on the image, $p_{i-1}$ 1242 and $p_i$ 1246, an optimal path finding algorithm finds the "best" path between the two points where "best" is

the one that has the least cost and bisects the. Thus $I_i^{in}$ pixel 1238 is the image intensity of the inside and dark pixel and $I_i^{out}$ 1234 is the image intensity outside and bright pixel along a path bisecting the $I_i^{in}$ 1238 and $I_i^{out}$ 1234 pixels and connecting pixels points $i$ 1246 and $i_{-1}$ 1242 adjacent to each side of the $I_i^{in}$ 1238 pixel.

(3) Previous Contour Distance Cost: When trying to find paths in 3D images, a constraint upon the paths on the current image are imposed and are close to a contour on a previous image. Therefore, a definition of a cost based on the distance of a point on the current path to a contour from a previous image can be made as according to equation E 1000:

$$C_c(p) = \sum_{i=p1}^{p2} D_i^P \qquad\text{E 16}$$

where $P$ is the previous contour and $D_i^P$ is the distance of point $i$ from the closest point on the previous contour. This distance can be easily evaluated using the Euclidean distance transform function computed for the previous contour.

A dynamic programming algorithm was used to find the least cost path between the initial edge points. The two steps typically involved in this are: (1) Computing the cost of the path from the starting point to each point on the image - this cost image (also known as the minimum action surface) using the fast-marching algorithms; (2) Locating the path connecting the end point to the starting point by gradient descent on the minimum action surface.

This multi-step segmentation algorithm of the present invention serves to establish a determination of the best path between two points and makes the segmentation much more robust. When the two points are quite far apart on the image, it still locates a good path that passes through the significant edges and completing the areas with missing edges.

Figure 14 image 1212 shows the optimal path found using this algorithm connecting the initial edge points.

The first image processed was the central image that had the fewest missing lateral boundaries. No previous contour constraint was used for this central image.

From this central image, the process was moved up to process all the images after the central image through the end of the sequence and then moved down to process all the images before the central image through the beginning of the sequence.

After the contours on all the images have been delineated, the volume of the segmented structure is computed. The contour inside the polygon is filled using a polygon filling algorithm to compute the volume. Then, the intensity value of a central pixel is determined using trilinear interpolation (where intensity values from six three-dimensional neighbors are used) to create a Cartesian volume from the polar coordinate images. Pixels on the boundary are assigned fractional values depending on the partial volume coverage. Finally, the sum of all the pixels in the reconstructed volume is multiplied by the voxel resolution to get the volume. The segmentation algorithm was made on images from a bladder phantom and on images of the bladder from several subjects. Boundaries were delineated on all of the images in the 3D dataset using the same center point for all images in the 3D sequence.

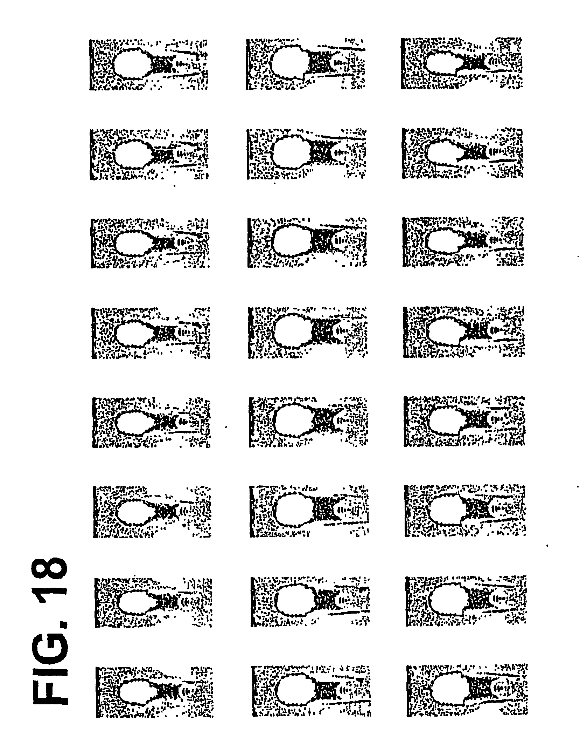

The segmentation algorithm was first tested on a bladder phantom with a known volume. From the bladder phantom 24 planes of data are acquired. The boundaries detected on this phantom are shown in Figure 18.

While the true volume of the bladder phantom is known to be 495 mL, the volume computed using our algorithm is 483 mL, or 97.6% accurate.

The accuracy for clinical measurements fared well. The segmentation algorithms as applied to 12 scan planes of real bladder images from five different subjects (3 females and 2 males) are shown in Table 1. The volume of urine was measured using a UroFlow device. The segmentation algorithm of the present invention was applied to images to measure volumes and compared to the urine volumes measured by the UroFlow device. The volume measured by the Uroflow device added to the post-Uroflow volume is considered the true bladder as a standard to compare to the volume determined by the segmentation algorithms applied to the 12 bladder scan images from each patient.

The table below shows the raw results on the five subjects:

Table 1: Bladder volume computational results

| Subject ID | True Bladder Volume (mL) | Segmentation Algorithm (mL) | Accuracy (%) |
|---|---|---|---|
| 102 | 391 | 367 | 93.9% |
| 130 | 238 | 232 | 97.5% |
| 138 | 651 | 642 | 98.6% |
| 148 | 469 | 434 | 92.5% |
| 162 | 169 | 104 | 61.5% |

Accuracy is defined as a % ratio and is calculated by taking the volume determined from the Segmentation Algorithm applied to the 12 image scans and dividing it by the True Bladder Volume, then multiplied by 100. The accuracy range of the 12 scan plane measurements to measured urine volumes is 61.5% to 98.6%. The median is 93.9%. The mean accuracy for the five measurements is 88.8%.

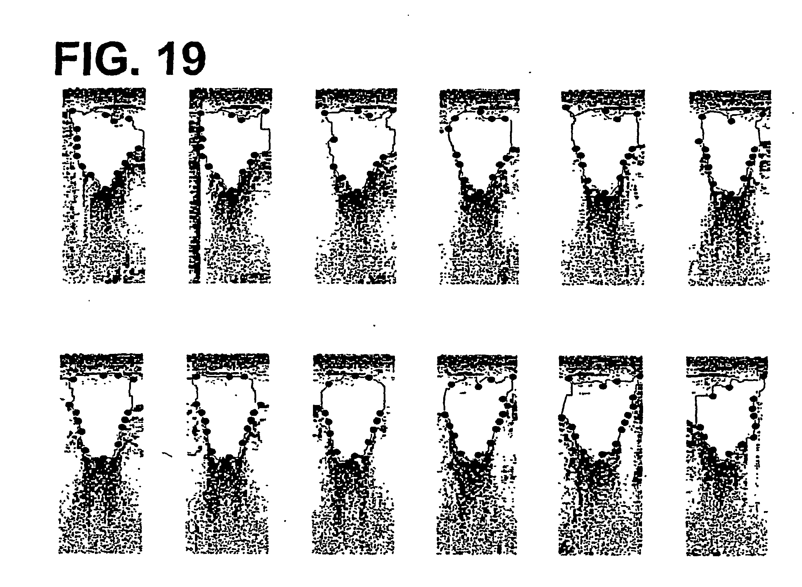

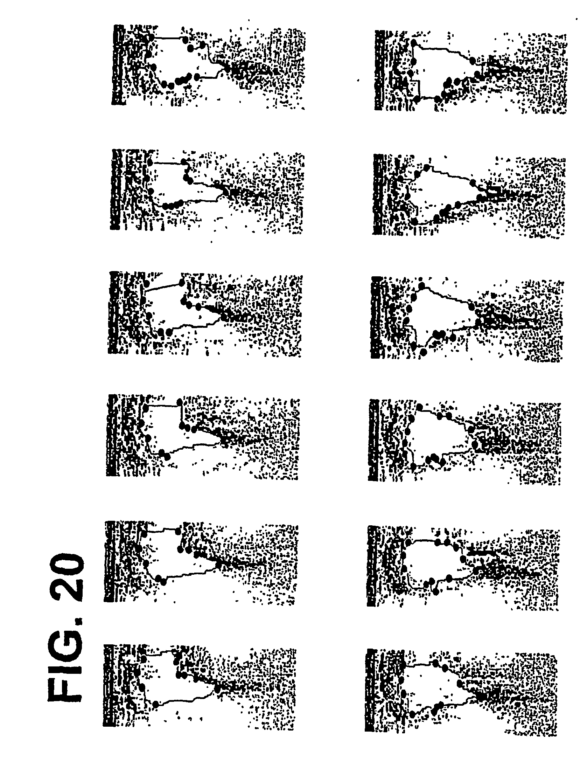

Figure 19 and Figure 20 respectively show the delineated bladder boundaries of 12 scan plane images for patients 138 and 148 subjected to the segmentation algorithm of the present invention. The segmentation algorithm set boundaries very reasonable boundaries except at a few planes in that the initial edge point is outside the expected location.

The segmentation algorithm of the present invention involves fitting minimum cost contours to known boundary points. The cost function is defined very similarly to snakes and other deformable models; however, instead of modifying an initial contour, our algorithm finds an optimal path between two known points on the boundary. The advantages compared to deformable models are: (a) The initial set of points needs to be very small and you do not need an entire initial contour, (b) The algorithm is more robust with respect to missing boundaries since missing boundaries are simply handled through an least cost path through them, (c) The algorithm is not iterative and always finds a unique global minimum for the cost function.

While the preferred embodiment of the invention has been illustrated and described, as noted above, many changes can be made without departing from the spirit and scope of the invention. For example, other uses of the invention include determining the areas and volumes of the prostate, heart, bladder, and other organs and body regions of clinical interest. Accordingly, the scope of the invention is not limited by the disclosure of the preferred embodiment.

**APPENDIX 1: EXAMPLES OF ALGORITHMIC STEPS**

**EXAMPLE IMAGE**

The Figure A below shows an example image on which some of the algorithmic steps are demonstrated. The size of this image is 20 rows by 20 columns.

Figure A: Example input image with 20 rows and
20 columns.

The image shown above is represented as a matrix of numbers and can also be displayed in 20 x 20 matrix form where the black pixels on the image have a number zero and white pixels have a number 10 in this case:

```
0   0   0   0   0   0   0   0   0   0   0   0   0   0   0   0   0   0   0   0
0   0   0   0   0   0   0   0   0   0   0   0   0   0   0   0   0   0   0   0
0   0   0   0   0   0   0   0   0   0   0   0   0   0   0   0   0   0   0   0
0   0   0   0   0   0   10  10  10  10  10  10  10  0   0   0   0   0   0   0
0   0   0   0   0   10  10  10  10  10  10  10  10  10  0   0   0   0   0   0
0   0   0   0   10  10  10  10  10  10  10  10  10  10  10  0   0   0   0   0
0   0   0   10  10  10  10  10  10  10  10  10  10  10  10  10  0   0   0   0
0   0   0   10  10  10  10  10  10  10  10  10  10  10  10  10  0   0   0   0
0   0   0   10  10  10  10  10  10  10  10  10  10  10  10  10  0   0   0   0
0   0   0   10  10  10  10  10  10  10  10  10  10  10  10  10  0   0   0   0
0   0   0   10  10  10  10  10  10  10  10  10  10  10  10  10  0   0   0   0
0   0   0   10  10  10  10  10  10  10  10  10  10  10  10  10  0   0   0   0
0   0   0   10  10  10  10  10  10  10  10  10  10  10  10  10  0   0   0   0
0   0   0   0   10  10  10  10  10  10  10  10  10  10  10  0   0   0   0   0
0   0   0   0   0   10  10  10  10  10  10  10  10  10  0   0   0   0   0   0
10  10  10  10  0   0   10  10  10  10  10  10  10  0   0   0   0   10  10  10
10  10  10  10  0   0   0   0   0   0   0   0   0   0   0   0   0   10  10  10
10  10  10  10  0   0   0   0   0   0   0   0   0   0   0   0   0   10  10  10
10  10  10  10  0   0   0   0   0   0   0   0   0   0   0   0   0   10  10  10
10  10  10  10  0   0   0   0   0   0   0   0   0   0   0   0   0   10  10  10
```

To illustrate some of our algorithms, we also use a noisy version of this example image. To create this image, we added random values to every pixel of the example image and this noisy image is shown in Figure B.

Figure B: Noisy image generated by adding random values to every pixel of the example image shown in Figure A.

As before, the noisy image is shown as a 20 x 20 matrix below:

```
1.4    4.1   1.1   1     3    0.074  2.7   0.37  3.8   0.91  4.5   0.67  3.3   0.52  4.2   2.6   4.6    2.8   0.86  1.7
2      0.83  3.5   2.3   4.7  1.4    3.1   0.97  3.3   2.5   4.7   0.11  4.3   0.79  4     3.6   2.8    3.8   0.65  2.8
2.5    2     2.6   0.41  1.4  4.1    3.4   1.9   4.4   2.1   1.7   1.3   2.8   2     3.5   2.6   3.3    3.9   1.1   0.59
3.6    2.6   4.7   4.3   4.4  4.9    13    11    11    13    12    11    15    2     2.3   3     3.9    2.4   0.53  0.85
1.5    3.6   3.6   2.8   0.51 10     14    14    12    13    12    10    14    10    0.41  4.8   0.53   4     0.71  1.4
0.56   2.8   1.1   1.6   10   14     10    12    11    15    11    14    11    15    14    4.1   0.0054 2.4   2.3   2.8
2.2    2.3   2.2   12    11   13     12    13    10    11    11    11    14    11    11    12    2.7    1     3.9   2.4
2.3    2.2   0.86  14    15   13     14    15    10    11    13    10    11    12    12    13    0.034  2.9   1.4   4.8
0.073  0.44  4.8   12    10   11     12    13    14    13    14    14    13    12    10    11    2.3    3.3   1.1   1.2
3.3    2.2   1.8   10    11   14     15    15    12    15    12    13    13    11    12    11    0.98   3.4   4.5   2.4
3.6    1.8   0.25  11    15   11     13    14    12    10    12    11    14    14    14    14    3.9    4.7   0.037 2.6
1.4    1.5   3.8   10    11   14     10    15    15    14    11    12    12    12    14    13    3.1    3.9   2.9   4
1.3    4.3   4.5   13    12   13     10    11    11    10    14    10    12    14    12    10    0.078  3.7   2.7   0.97
3.5    3.8   1.4   0.61  11   11     10    14    14    13    13    14    12    12    14    2.8   4.5    4.3   3.3   4.5
3.9    4.7   1.3   2.6   3.4  13     11    15    13    11    11    14    13    12    2.4   2.3   3.8    5     1.6   4.6
15     13    15    11    4.8  3      13    11    14    14    15    11    11    2.5   2.8   4.5   4.5    13    11    10
12     10    11    14    3.8  3.3    2.3   0.61  2.4   1.3   3.2   2.8   0.01  0.85  3.1   1.4   3.8    13    12    14
15     13    15    12    3.3  0.92   3.5   3.8   5     4.7   1.1   0.8   4     2.6   3.3   0.33  1.9    14    11    15
12     14    14    14    0.65 3.2    2.9   3.6   1.9   0.69  3.4   3     2.6   3.2   3.1   2.4   1.7    12    13    14
14     15    14    10    0.48 0.85   2.5   3.3   2.7   2.6   3.3   1.7   1.1   0.081 3.4   4.9   2.5    13    15    15
```

**HEAT FILTER - 514**

The Laplacian (second derivative) of the example image is computed as the sum of the second partial x-derivative and second partial y-derivative of the image: $\dfrac{\partial^2 u}{\partial x^2} + \dfrac{\partial^2 u}{\partial y^2}$. This Laplacian is the right-hand side of the heat equation E1.

The Laplacian of the example image is computed and this is shown in Figure C(1). Notice the negative values on the inside of the bright areas and positive values on the outside of the bright areas at the edges of the regions. The Laplacian is zero an pixels far from edges.

(1)                                    (2)

Figure C (1): The Laplacian of the example image
shown in Figure A. (2) The Laplacian added back
to the input image.

When this Laplacian is added back to the input image, after multiplying with a step size parameter, it results in blurring of the edges since the bright areas are reduced in brightness and the dark areas are increased in brightness - this output is shown in Figure C(2). This is the output of a single iteration of the heat filter. The heat filter outputs after 4 iterations and after 20 iterations are shown in (1) (2)

Figure D. Notice the progressive blurring of the image as you apply more iterations.

(1)                                    (2)

Figure D: Heat filter outputs after 4 iterations (1)
and after 20 iterations (2) on the image shown in
Figure A.

Applying 20 iterations of the heat filter to the noisy image from Figure B results in a reduction of noise and a blurring of the image. This output is shown in Figure E.

Figure E: The result of applying heat filter to a noisy image.

Note that in this image, the noise has been significantly removed, although the image is very blurred. This image looks very similar to the heat filtered version of the noiseless image shown in Figure D(2).

**SHOCK FILTER - 518**

The application of the shock filtered to the blurred image is shown in Figure E to sharpen this image. For purposes of the shock filter, the Laplacian of an image (Equation E4) is computed as:

$$\ell(u) = u_{xx}u_x^2 + 2u_{xy}u_xu_y + u_{yy}u_y^2.$$

This is computed for the blurred image and is shown below in shown in Figure F(1). Again, notice the negative values on the inside of the bright areas and positive values on the outside of the bright areas at the edges of the regions.
The gradient magnitude of the blurred image (Equation E3) is:

$$\|\nabla u\| = \sqrt{u_x^2 + u_y^2}$$

This is computed for the blurred image and is shown in Figure F(2) below. Note that the gradient magnitude is highest at the edges and zero in smooth regions.

(1)

(2)

Figure F (1) The Laplacian of the blurred image.
(2) The gradient magnitude of the blurred image.

Using a threshold value, t, on pixel gradient to be 0.7 we can compute the F function, which is positive where the Laplacian is greater than zero and the gradient magnitude is greater than a threshold, negative where the Laplacian is less than zero and the gradient magnitude is greater than the threshold and zero elsewhere:

$$F(\ell(u)) = 1, \text{ if } \ell(u) > 0 \text{ and} \|\nabla u\| > t$$
$$= -1, \text{ if } \ell(u) < 0 \text{ and} \|\nabla u\| > t$$
$$= 0, \text{ otherwise}$$

This F image is shown in Figure G(1) below. Note that as with the Laplacian image, everything outside the original white regions at the edge is 1, everything inside the original white regions at the edge is -1. The boundaries of the original region can be seen as the transition between the negative and the positive F values (the zero crossings).

Now, the right hand side of the shock filter equation E2, is the negative of the product of the F function image Figure G(1) and the gradient magnitude image shown in Figure F(2): $- F(\ell(u)) \|\Delta u\|$

This right hand side of the shock filter equation is shown in Figure G(2). If we add this image to the original blurred image we can see that it has the opposite effect of the heat filter. The dark pixels in Figure G(2) are subtracted from the blurred image and the bright pixels are added to the blurred image basically, thereby making the blurred image more crisp. The addition of this image to the input blurred image is basically what constitutes a single iteration of the shock filter - this addition is shown in Figure H(1).

After 20 iterations, a crisper output as shown in Figure H(2) is obtained. While this image is not identical to the input noiseless image of Figure A, it is quite similar to it and much more noise free as compared to the noisy version shown in Figure B.

(1)                                                    (2)

Figure G: (1) The F function computed from the Laplacian and the gradient. (2) The negative of the product of the F function and the gradient.

Figure H(1) The output of 1 iteration of the shock filter on the blurred image from Figure E. (2) The output of 20 iterations of the shock filter on the same blurred image.

## INTENSITY CLUSTERING - 422

In intensity clustering, the enhanced image is subject to the k-means clustering algorithm. In this example, the enhanced image, shown in Figure H(2), is clustered into 4 clusters.

The minimum and maximum intensity values are 1.69 and 13.40. We divide that range into 4 partitions and get the following initial cluster boundaries.

1.19 4.12 7.04 9.98 13.90

With this partition, all pixels with values between 1.19 and 4.12 are assigned into cluster 1, all between 4.12 and 7.04 are assigned into cluster 2, etc. This initial assignment of every pixel to the 4 clusters is shown in Figure I(1). Based on this assignment new cluster centers are found and new partition boundaries found accordingly - after this first iteration, the cluster boundaries move slightly and they are now at:

1.19 4.08 6.30 9.57 13.90.

In the next iteration, the pixel assignment based on these partition boundaries is shown in Figure I(2). Finally, after 6 iterations, the partition boundaries change very little between iterations and are set to:

1.19 3.65 5.646 9.25 13.90

The final assignment of pixels to the 4 clusters is shown in Figure I(3). For this example, the output of the clustering step is the set of pixels assigned to the brightest cluster. This output is shown in Figure I(4).

(1)

(2)

(3)

(4)

Figure I: The output of clustering at different iterations of the k-means clustering process. (1) Initial assignment (2) After the first iteration (3) Final assignment after 6 iterations. (4) The one

brightest cluster representing the region of interest.

**SPATIAL GRADIENT - 526**

To compute the gradient magnitude of the enhanced image shown in Figure H(2), the x-derivative $u_x$ and the y-derivative $u_y$ of the image is calculated and then the sum of squares of the two images is calculated to get the gradient magnitude -- $\|\nabla u\| = \sqrt{u_x^2 + u_y^2}$. The x- and y-derivatives and the gradient magnitudes of the enhanced image are shown in Figure J.

(1)                          (2)                          (3)

Figure J: Spatial gradients of the image shown in Figure H(2): (1) x-derivative of image (2) y-derivative of image (3) gradient magnitude of image.

## HYSTERESIS THRESHOLD - 530

In the hysteresis threshold, two threshold values based on the percentage of non-edge pixels desired and the ratio of the two thresholds is determined. In this example, the percentage non-edge pixels desired is set to 90% and the lower to upper threshold ratio is set to 0.5.

The upper threshold is first computed by looking at the histogram and the cumulative histogram of the gradient magnitude image shown in Figure J(3). The histogram of the gradient magnitude image is shown in Figure K(1). The histogram shows the count of pixels of different gradient values on the image. The cumulative histogram calculated by cumulatively adding up the bins of the histogram is shown in Figure K(2). The cumulative histogram shows the count of all pixels less than a given value in the gradient magnitude image. The horizontal line shown in Figure K(2) corresponds to a y-value of 90% of the image pixels (0.90 * 20 * 20 = 360). The intersection of this line with the cumulative histogram gives the threshold at which 90% of the image pixels will be marked as non-edge and 10% will be marked as edge. From the graph, we can see that a threshold of 7 satisfies that condition. The lower threshold is set to 0.5 times the upper threshold of 7, which gives 3.5.

Figure K: Histogram and cumulative histogram of the gradient magnitude image.

For the hysteresis threshold, the threshold of the image at the two threshold values of 3.5 and at 7 is set and then all edge segments from the lower threshold image is selected that have at least one higher threshold pixel. The two thresholded images and the output of the Hysteresis threshold step are shown in Figure L. In this example, note that one edge segment - (row 15, column 2) has no pixel greater than the higher threshold and is therefore removed from the output. All other edge segments from Figure L(1) are retained in the output.

(1)                                    (2)                                    (3)

Figure L: (1) Gradient image thresholded at 3.5 — all pixels greater than 3.5 are shown as white (2) gradient image thresholded at 7, (3) Output of hysteresis threshold.

### MATCHING EDGES FILTER - 538

The matching edges filter selects pairs of matching edges from among all the edges found by the hysteresis threshold step (Figure L(3)). The y-derivative image , shown in Figure J(2) is used in this step to help find the vertical matching edges. The matching edges found by this step of the algorithm are shown in Figure M(1) and the filled region between the leading the trailing edges are shown in Figure M(2). Note that this step gets rid of edges of regions on the periphery of the image since it cannot find matching trailing edges for those regions. Also, note that in the output filled image we have some unfilled lines where the gradient was primarily horizontal or where the matching edges did not exist.

(1)                                                              (2)

Figure M: (1) Matching edges found by the algorithm – leading edges shown in gray and lagging edges shown in white. (2) The region

between the leading the lagging edges filled by white pixels.

**AND IMAGES - 442**

The two images shown in Figure I(4) and Figure M(2) represent the segmentation of the input noisy image shown in Figure B. Neither of them represents a perfect output. The region-based algorithm finds extraneous regions while the edge-based algorithm misses part of the desired region. The two images are combined using a Boolean AND operator and the output is shown in Figure N.

Figure N: Combining the region-based and edge-based segmentation.

**CLOSE AND OPEN - 546 & 550**

Using a structuring element shaped like a line of width 5 pixels, we close the output of AND images and this gives us a single region representing the structure of interest as shown in Figure O.

Figure O: Result of closing the image from Figure N.

Opening this image does not change the result - however, Figure P shows an example of opening on another image. The input image, Figure P(1), contains two regions, one large region and one small region. The large region is 10 pixels in width and height while the small region is 4 pixels in width and height. Using a structuring element shaped like a line of length 5 pixels and applying morphological opening to the input image results in an image shown in Figure P(2) where the smaller region whose width was smaller than the structuring element was removed from the image.

(1)                                    (2)

Figure P: (1) Input image containing two foreground regions. (2) Output image after opening where the smaller region was removed because it was smaller than the structuring element.

**FILTER DEEP REGIONS - 554**

This filtering step is used to remove regions from a segmented image that start at a location deeper than a user specified depth threshold. This filtering is illustrated in Figure Q, where the input image contains two regions -- one region starts at row 2 and the other region starts at row 14. For each region on the input image, the starting row is determined after doing a connected component labeling - then using a depth threshold of 12, the regions where the starting row depth is greater than the depth threshold are removed from the input image.

Figure Q: (1) Input image containing two foreground regions. (2) Output image after removing the region whose starting row is greater than the depth threshold of 12.

**HEAD DIAMETER RANGE - 730**

For a gestational age of 20 weeks, the bi-parietal diameter (BPD) is typically 46 mm. The calculated Head Diameter search range, which ranges from -30% to +30% of the typical BPD value, is found to be:
32.2 36.8 41.4 46.0 50.6 55.2 59.8
For a gestational age of 36 weeks, the bi-parietal diameter (BPD) is typically 88 mm. The calculated Head Diameter search range, which ranges from -30% to +30% of the typical BPD value, is found to be:
61.6 70.4 79.2 88.0 96.8 105.6 114.4

**POLAR HOUGH TRANSFORM - 738**

To illustrate the polar Hough transform, an exemplary image is generated of a circle in the Cartesian coordinates using the circle equations, E5. This circle image was then dilated to result in a disk like shape and then a few rows of data were set to zero. This input image is shown in Figure R(1). The goal of the circular Hough transform is to find the circle that best fits the white pixels in this input image.
Next, this Cartesian input image was converted to polar domain by inverse scan conversion and the resulting image is shown in Figure R(2). To this polar coordinate image, the polar Hough transform for a circle was applied, where for each white pixel on the image a polar circle of a known radius was drawn. The output of the Hough transform is the sum of all such circles and this is shown in Figure R(3). The center of the best-fit circle has the most points passing through it. So, to determine the best-fit circle, we simply found the pixel on the Hough output with the maximum value. The point with the maximum value represents the center of the best-fit circle.

(1)                    (2)                (3)

Figure R: (1) The input Cartesian coordinate partial circle (2) The partial circle converted to polar coordinates, (3) The Hough transform output showing the sum of polar circles drawn around each white pixel of the input image.

The best-fit circle determined by this Hough transform procedure is overlaid on the polar coordinate image and the Cartesian coordinate image and the two drawings are shown in Figure S.

Figure S: The best fit circle found using a Polar coordinate Hough transform (1) overlaid on the polar coordinate input data and (2) overlaid on the Cartesian coordinate input data.

**FILL CIRCLE REGION - 746**

A circle region in Cartesian space has a somewhat different shape in the Polar coordinate space. To illustrate this polar coordinate circle, we use both Cartesian coordinate and polar coordinate circles. First, a Cartesian coordinate circle of radius 50 pixels and a center (120,250) is drawn using the circle equation, E5, $(x\text{-}x_0)^2 + (y\text{-}y_0)^2 = R^2$ -- this circle is shown in Figure T(1).

Now, the Cartesian center coordinates $(x_0, y_0)$ are converted from Cartesian to polar center $(r_0, \phi_0)$ using the scan conversion equations:

$$r_0{}^2 = (x_0{}^2 + y_0{}^2),$$

$$\phi_0 = \tan^{-1}(y_0 / x_0)$$

Next, the polar coordinate circle drawing and filling equation, E6, $(r\sin\phi - r_0\sin\phi_0)^2 + (r\cos\phi - r_0\cos\phi_0)^2 = R^2$, is applied to draw and fill the same circle of radius R=50, in polar coordinates. This circle in the polar coordinates is shown in Figure T(2).

Finally, to verify that this polar coordinate circle in indeed a representation of the Cartesian coordinate circle, the polar circle image is scan-converted to generate the representative Cartesian coordinate circle image. This scan-converted circle is shown in Figure T(3). Comparing Figure T(1) and Figure T(3), it can be seen that while the two circles are not identical, they are fairly similar. The differences are due to the fact that the polar coordinate data cannot represent Cartesian data completely.

(1)         (2)         (3)

Figure T Polar coordinate circle filling. (1) A filled circle in a Cartesian coordinate image (2) The equivalent filled circle in polar coordinate image, (3) The scan converted polar coordinate filled circle image.

APPENDIX 2: MATLAB SOURCE CODE

## HEAT FILTER – 514

```
function out = heat_flow2D( I, num_iter, time_step )
% heat_flow2D: 2D heat flow filter same as Gaussian filtering.
%
% out = heat_flow2D( I, num_iter, time_step )
% * I is the input image
% * num_iter is the number of iterations for which to run the filter
% * time_step is the size of the time step between iterations.
% * out is the output smoothed image
%
% The heat flow equation is I(t+1) = I(t) + time_step * Laplacian(I)
% Gaussian standard deviation (sigma) = sqrt( 2 * num_iter * time_step )
% or, if time_step = 0.25, num_iter = 2 * sigma^2.

[m n] = size(I);
update = zeros(m, n);
rows = 1:m;
cols = 1:n;

prev_rows = rows - 1;
prev_rows(1) = 1;
next_rows = rows + 1;
next_rows(m) = m;

prev_cols = cols - 1;
prev_cols(1) = 1;
next_cols = cols + 1;
next_cols(n) = n;

for iter = 1:num_iter
    update = (I(rows, next_cols) + I(next_rows,cols) - 4*I(rows,cols) + I(prev_rows, cols)
+ I(rows, prev_cols))/4;
    I = I + time_step * update;
end

out = I;
```

## SHOCK FILTER - 518

```
function out = shock_filter_grad2D( I, num_iter, time_step, grad_thresh )
%-- 2D shock filter
%-- the equation is I(t+1) = I(t) - sign(Ixx) . | grad( I ) |
%-- sign(Ixx) is the laplace and grad(I) is the updwind derivative
% here instead of using Ixx for edges, we use magnitude of grad(I) as edge.

[m n] = size(I);
update = zeros(m, n);
```

```
diff = zeros(m,n);
gradmat = zeros(m, n);
gradmag = zeros(m, n);
laplace  = zeros(m, n);
diffpos = zeros(m, n);
diffneg = zeros(m, n);
dplus = zeros( m, n);
dminus = zeros( m, n);
cond1 = zeros( m, n);
cond2 = zeros( m, n);

rows = 1:m;
cols = 1:n;

prev_rows = rows - 1;
prev_rows(1) = 1;
next_rows = rows + 1;
next_rows(m) = m;

prev_cols = cols - 1;
prev_cols(1) = 1;
next_cols = cols + 1;
next_cols(n) = n;


for iter = 1:num_iter
    %laplace = (I(rows, next_cols) + I(next_rows,cols) - 4*I(rows,cols) + I(prev_rows,
cols) + I(rows, prev_cols))/4;
    laplace = laplace2( I );

    % instead of centered diff - lets use upwind
    %gradmat = 0.5*abs(I(next_rows, cols) - I(prev_rows, cols));

    %row derivative forward
    diff = I(next_rows,cols) - I(rows,cols);
    cond1 = diff >= 0;
    cond2 = diff < 0;
    diffpos( cond1 ) = diff( cond1 );
    diffpos( cond2 ) = 0;
    diffneg( cond2 ) = diff( cond2 );
    diffneg( cond1 ) = 0;

    dplus  = diffneg .* diffneg;
    dminus = diffpos .* diffpos;

    %row derivative backward
    diff = I(rows,cols) - I(prev_rows,cols);
    cond1 = diff >= 0;
    cond2 = diff < 0;
    diffpos( cond1 ) = diff( cond1 );
```

36

```
diffpos( cond2 ) = 0;
diffneg( cond2 ) = diff( cond2 );
diffneg( cond1 ) = 0;


dplus  = dplus + diffpos .* diffpos;
dminus = dminus + diffneg .* diffneg;


%col derivative forward
diff = I(rows,next_cols) - I(rows,cols);
cond1 = diff >= 0;
cond2 = diff < 0;
diffpos( cond1 ) = diff( cond1 );
diffpos( cond2 ) = 0;
diffneg( cond2 ) = diff( cond2 );
diffneg( cond1 ) = 0;


dplus  = dplus + diffneg .* diffneg;
dminus = dminus + diffpos .* diffpos;


%col derivative backward
diff = I(rows,cols) - I(rows,prev_cols);
cond1 = diff >= 0;
cond2 = diff < 0;
diffpos( cond1 ) = diff( cond1 );
diffpos( cond2 ) = 0;
diffneg( cond2 ) = diff( cond2 );
diffneg( cond1 ) = 0;


dplus  = dplus + diffpos .* diffpos;
dminus = dminus + diffneg .* diffneg;


dplus = sqrt( dplus );
dminus = sqrt( dminus );


gradmat( laplace >= 0 ) = dplus( laplace >= 0 );
gradmat( laplace < 0 )  = dminus ( laplace <  0 );


gradmat(gradmat < grad_thresh) = 0;


laplace( laplace > 0 ) = 1;
laplace( laplace < 0 ) = -1;


update = -gradmat .* laplace;
I = I + time_step .* update;
end


out = I;
```

## INTENSITY CLUSTERING – 422

```
function min_img = cluster_image( in_img, num_clusters, sample_factor, num_iterations
)
% cluster_image: Cluster an intensity image using the k-means algorithm.
%
% out_img = cluster_image( in_img, num_clusters, sample_factor, num_iterations )
%    * in_img is the input intensity image to be clustered
%    * num_clusters is the number of output clusters desired
%    * sample_factor is a factor by which to downsample the input data. If 1, then no
sampling, if 2, then reduces
%      input data by half.
%    * num_iterations is the maximum number of iterations that the algorithm should be
run if it does not converge.
%      usually it converges in about 2-6 iterations - so you can set this to be about 20.
%    * min_img is the output image consisting of pixels lying on the cluster with the
minimum mean.
%
%   A simple k-means algorithm is used for this clustering.

convergence_threshold = 0.05;

in_data = downsample( in_img(:), sample_factor );

minval = min( in_data );
maxval = max( in_data );

incval = ( maxval - minval )/num_clusters;

%cluster boundaries
boundaries = zeros(1, num_clusters + 1 );
boundaries(1) = (minval-0.5);
boundaries(num_clusters+1) = (maxval+0.5);
for i = 2:num_clusters
   boundaries(i) = boundaries(i-1) + incval;
end

a = 1; b = [0.5 0.5];
centers = zeros( 1, num_clusters );
centers = filter( b, a, boundaries );
centers = centers( 2:end );
old_centers = centers;

for iter = 1:num_iterations

   %lets quantize the data according to the boundaries
   for j = 1:num_clusters
      cdata = in_data( in_data <= boundaries(j+1) & in_data > boundaries(j) );
      centers(j) = mean(cdata);
```

```
end

%lets recompute the boundaries
newb = filter( b, a, centers );
newb( 1 ) = (minval-0.5);
boundaries = [newb, (maxval+0.5)];

%centers
diff = norm( ( centers - old_centers ) ./ old_centers );
if ( diff <= convergence_threshold )
    %disp(sprintf('converged in %d iterations', iter ));
    break;
end

old_centers = centers;
end

min_thresh1 = boundaries( 1 );
min_thresh2 = boundaries( 2 );
min_img = ( in_img <= min_thresh2 ) & ( in_img > min_thresh1 );

min_img = reshape( min_img, size( in_img ) );
```

### SPATIAL GRADIENTS – 526

```
function [xgrad,ygrad,grad_mag] = spatial_gradients( in_img )
% spatial_gradients: Compute spatial gradients of the 2D input image
%
% [xgrad,ygrad,grad_mag] = spatial_gradients( in_img )
%    * in_img is the input input image
%    * xgrad is the x-direction gradient
%    * ygrad is the y-direciton gradient
%    * grad_mag is the gradient magnitude
%

kernx = [-1 1];
kerny = [-1; 1];

xgrad = imfilter( in_img, kernx );
ygrad = imfilter( in_img, kerny );
grad_mag = sqrt( xgrad .^ 2 + ygrad .^ 2 );
```

### HYSTERESIS THRESHOLD – 530

```
function out = hysteresis_threshold( grad_mag, PercentNonEdge, FractionLow )
% hysteresis_threshold: Perform hysteresis thresholding by automatically computing two
thresholds
%
% out = hysteresis_threshold( grad_mag, PercentNonEdge, FractionLow )
```

```
%   * grad_mag is gradient magnitude image to be thresholded
%   * PercentNonEdge is the percentage of non-edge pixels desired (0.97 works good)
%   * FractionLow is the ratio between the lower and the upper threshold (0.5 works
good)


%lets try to figure out the thresholds
maxgrad = max( grad_mag(:) );
[c x] = hist( grad_mag(:), ceil( maxgrad ) );
[m n] = size( grad_mag );
high_thresh = min( find( cumsum( c ) > PercentNonEdge * m * n ) );

%lets threshold the gradients
out = hysteresis_threshold_core( grad_mag, FractionLow * high_thresh, high_thresh );


function out = hysteresis_threshold_core( img, t1, t2 )
%do a hystersis theresholding of an image at a lower threshold of t1 and an upper
threshold of t2
%basically, we first do a thresholding at the lower value
%then we do a connected component
%then we pick those components which have at least one value above t2
%here, we are going to do it a little differently,
%   1. we will first threshold at t2
%   2. we will select one point each from t2
%   3. then we will threshold at t1
%   4. for each point from t2, we will use bwlabel/bwselect to find the component on the
t1 image
%


%lets first threshold at higher threshold t2
t2_thresh = img > t2;


[r c] = find( t2_thresh );


%lets now threshold at lower thresh t1
t1_thresh = img > t1;


out = bwselect( t1_thresh, c, r, 8 );
```

## MATCHING EDGES FILTER - 538

```
function [out_filled, out_edg2] = find_matching_vertical_edges( in_edg, ygrad )
% find_matching_vertical_edges: Find mataching edges in the vertical direction
%
% out_filled = find_matching_vertical_edges( in_edg, ygrad )
%   * in_edg is the input edge image
%   * ygrad is the y-direction gradient
%   * out_filled is the output filled region between edges
```

```
%

out_ygrad = ygrad;
out_ygrad( in_edg == 0 ) = 0;

dist_thresh = 5;

%for every line, select the closest edge pairs
[npts nline] = size( in_edg );
out_edg2 = zeros( size( in_edg ) );
out_filled = out_edg2;

for line = 1:nline
    fw_pts = find( out_ygrad(:,line) < 0 );
    bw_pts = find( out_ygrad(:,line) > 0 );
    lfw = length( fw_pts );
    lbw = length( bw_pts );
    if ( lfw == 0 | lbw == 0 )
        continue;
    end
    if ( ( lfw == 1 ) & ( lbw == 1 ) )
        if ( bw_pts(1) > fw_pts(1) )
            out_edg2( fw_pts(1), line ) = 1;
            out_edg2( bw_pts(1), line ) = 2;
            out_filled( fw_pts(1):bw_pts(1), line ) = 1;
        end
        continue;
    end

    votes_fw = zeros( size( fw_pts ) );
    votes_bw = zeros( size( bw_pts ) );

    %for every front wall point find the closest backwall point
    for fw = 1:lfw
        curr_fw = fw_pts( fw );
        min_bw = 0;
        min_dist = 1000000;
        for bw = 1:lbw
            curr_dist = bw_pts( bw ) - curr_fw;
            if ( curr_dist > dist_thresh & curr_dist < min_dist )
                min_bw = bw;
                min_dist = curr_dist;
            end
        end
        if ( min_bw > 0 )
            votes_fw( fw ) = votes_fw( fw ) + 1;
            votes_bw( min_bw ) = votes_bw( min_bw ) + 1;
        end
    end
```

```
%for every bacak wall point find the closest frontwall point
for bw = 1:lbw
   curr_bw = bw_pts( bw );
   min_fw = 0;
   min_dist = 1000000;
   for fw = 1:lfw
      curr_dist = curr_bw - fw_pts( fw );
      if ( curr_dist > dist_thresh & curr_dist < min_dist )
         min_fw = fw;
         min_dist = curr_dist;
      end
   end
   if ( min_fw > 0 )
      votes_fw( min_fw ) = votes_fw( min_fw ) + 1;
      votes_bw( bw ) = votes_bw( bw ) + 1;
   end
end

%at this point, the fw and bw with votes greater than 1 should be matching
good_fw_pts = fw_pts( votes_fw > 1 );
good_bw_pts = bw_pts( votes_bw > 1 );

if ( length( good_fw_pts ) ~= length( good_bw_pts ) )
   disp('match not found' );
else
   for i = 1:length( good_fw_pts )
      out_edg2( good_fw_pts(i), line ) = 1;
      out_edg2( good_bw_pts(i), line ) = 2;
      out_filled( good_fw_pts(i):good_bw_pts(i), line ) = 1;
   end
end

end
```

## CLOSE & OPEN – 546 & 550

```
function out = close3D_brick( in, size1, size2, size3 )

      tmp = dilate3D_brick(in, size1, size2, size3 );
      out = erode3D_brick(in, size1, size2, size3 );

function out = open3D_brick( in, size1, size2, size3 )

      tmp = erode3D_brick(in, size1, size2, size3 );
      out = dilate3D_brick(in, size1, size2, size3 );

function out = erode3D_brick( in, size1, size2, size3 )
      % erode3D_brick: Erode a binary image with a brick shaped structuring element
      %
      % out_img = erode3D_brick( in, size1, size2, size3 )
```

```
%    * in_img is the input binary image to be dilated
%    * size1 is the size of the brick along the row dimension
%    * size2 is the size of the brick along the column dimension
%    * size3 is the size of the brick along the slice dimension
%
% A seprable order-statistic filter with the ordinal as min is used.

[nr nc np] = size( in );
out = in;

for p = 1:np
    %lets first do the dilation along rows
    out(:,:,p) = ordfilt2( out(:,:,p), 1, ones( size1, 1 ) );

    %lets first do the dilation along columns
    out(:,:,p) = ordfilt2( out(:,:,p), 1, ones( 1, size2 ) );
end

%now lets do it along the third dimension
%first permute dimension
tmp = permute( out, [3, 2, 1] );
for r = 1:nr
    tmp(:,:,r) = ordfilt2( tmp(:,:,r), 1, ones( size3, 1 ) );
end

out = permute( tmp, [3, 2, 1] );

function out = dilate3D_brick( in, size1, size2, size3 )
    % dilate3D_brick: Dilate a binary image with a brick shaped structuring element
    %
    % out_img = dilate3D_brick( in, size1, size2, size3 )
    %    * in_img is the input binary image to be dilated
    %    * size1 is the size of the brick along the row dimension
    %    * size2 is the size of the brick along the column dimension
    %    * size3 is the size of the brick along the slice dimension
    %
    % A seprable order-statistic filter with the ordinal as max is used.

    [nr nc np] = size( in );
    out = in;

    for p = 1:np
        %lets first do the dilation along rows
        out(:,:,p) = ordfilt2( out(:,:,p), size1, ones( size1, 1 ) );

        %lets first do the dilation along columns
        out(:,:,p) = ordfilt2( out(:,:,p), size2, ones( 1, size2 ) );
    end

    %now lets do it along the third dimension
```

```
%first permute dimension
tmp = permute( out, [3, 2, 1] );
for r = 1:nr
    tmp(:,:,r) = ordfilt2( tmp(:,:,r), size3, ones( size3, 1 ) );
end


out = permute( tmp, [3, 2, 1] );
```

## FILTER DEEP REGIONS – 554

```
function out = filter_deep_regions( in, depthThreshold )
% filter_deep_regions: Filter out regions that start beyond the depth threshold
%
%  out = filter_deep_regions( in, depthThreshold )
%    * in is the input binary image
%    * depthThreshold is the threshold beyond which regions are not acceptable
%    * out is the output filtered binary image


[ccmp n] = bwlabel( in );
props = regionprops( ccmp, 'BoundingBox' );
out = zeros( size(in) );
j = 0;
for i = 1:n
    if ( props(i).BoundingBox(2) < depthThreshold )
        out = out + ( ccmp == i );
        j = j+1;
    end
end
```

## HEAD DIAMETER RANGE – 730

```
function rad_range = radrange_lookup( age, imgParams )
%lookup the radius range in pixels given the gestational age and the
%resolution


mean_bpd = bpd_lookup( age );
mean_rad = 0.5 * mean_bpd / imgParams.AxialResolution;
rad_range = [mean_rad - ( 0.3 * mean_rad )
            mean_rad - ( 0.2 * mean_rad ),
            mean_rad - ( 0.1 * mean_rad ),
            mean_rad,
            mean_rad + ( 0.1 * mean_rad ),
            mean_rad + ( 0.2 * mean_rad )
            mean_rad + ( 0.3 * mean_rad )];


function bpd_out = bpd_lookup( age )
    %lookup the bpd in mm given the gestational age
```

44

```
%BPD Table   wks; BPD in mm
bpd_table = [14, 26;
        15, 30;
        16, 33.5;
        17, 36.5;
        18, 40;
        19, 43;
        20, 46;
        21, 49.5;
        22, 52.5;
        23, 55.5;
        24, 58;
        25, 61;
        26, 64;
        27, 66.5;
        28, 69;
        29, 72;
        30, 74;
        31, 76.5;
        32, 79;
        33, 81;
        34, 83.5;
        35, 86;
        36, 88;
        37, 90;
        38, 92;
        39, 94;
        40, 96;
    ];

bpd_out = 0;
if ( age < 14 )
   return;
end

bpd_out = 100;
if ( age > 40 )
   return;
end

index = round( age - 14 + 1 );
bpd_out = bpd_table(index,2);
```

## HEAD EDGE DETECTION – 734

```
function edg2 = find_head_regions( in_edg, ygrad, wall_edg, region_img, radrange )
%use ygrads to find head regions

IntenThresh = 15;
DistThresh = 12;
```

```
ygrad( in_edg == 0 ) = 0;


[npts nline] = size( ygrad );
edg2 = wall_edg;

%lets go over each line and find potential head locations
%potential head locations will have
%   a. good front wall and back wall pairs identified by the wall_edg input
%   b. a matching positive gradient before the front wall - within a short distance
%   c. a matching negative gradient after the back wall - within a short distance
for line = 1:nline

   neg_grad_pts = find( ygrad(:,line) < 0 );
   pos_grad_pts = find( ygrad(:,line) > 0 );
   ln = length( neg_grad_pts );
   lp = length( pos_grad_pts );

   fw = find( wall_edg(:,line) == 1 );
   bw = find( wall_edg(:,line) == 2 );
   lfw = length(fw);
   lbw = length(bw);

   if ( lfw > 0 & lbw > 0 & lfw == lbw )
      for i = 1:lfw
         dist =  bw(i) - fw(i);
         %if distance is beyond the rad range, set to zero
         if ( dist < 2*radrange(1) | dist > 2*radrange(end) )
            edg2(fw(i),line) = 0;
            edg2(bw(i),line) = 0;
            continue;
         end

         %if mean intensity is not at least 80% dark
         if ( mean( region_img(fw(i):bw(i), line ) ) < 0.8 )
            edg2(fw(i),line) = 0;
            edg2(bw(i),line) = 0;
            continue;
         end

         %if within DistThresh of front wall is not a postive gradient point - else expand
         diffs = fw(i) - pos_grad_pts;
         if ( sum( diffs > 0 & diffs < DistThresh ) == 0 )
            edg2(fw(i),line) = 0;
         else
            if ( fw(i) > 3 & fw(i) < npts - 3 )
               edg2( (fw(i)-3):(fw(i)+3), line ) = 1;
            end
         end
```

```
%if within DistThresh of back wall is not a negative gradient point
diffs = neg_grad_pts - bw(i);
if ( sum( diffs > 0 & diffs < DistThresh ) == 0 )
    edg2(bw(i),line) = 0;
else
    if ( bw(i) > 3 & bw(i) < npts - 3 )
        edg2( (bw(i)-3):(bw(i)+3), line) = 2;
    end
end
    end
else
    edg2(:,line) = 0;
end
end
```

## POLAR HOUGH TRANSFORM – 738

```
function out = hough_circle_polar( edg_img, radii, xducerOffset, phiAngles )
%compute a hough transform for circle finding on a pre-scan converted image
%the function goes throgh every edge pixel on the image and adds an accumulator
corresponding to every radii

nradii = length( radii );
[nrow ncol] = size( edg_img );

out = zeros( nrow, ncol, nradii );

angles = 0:18:360;
angles = pi*angles/180;
cosa = cos( angles );
sina = sin( angles );

prows = cosa;
pcols = cosa;

len = length( angles );
hlen = len / 2;

%go over every edge pixel
for row = 1:nrow
    for col = 1:ncol
        curr_edg = edg_img( row, col );
        if ( curr_edg > 0 )
            for rad = 1:nradii
                [prows, pcols] = draw_polar_circle( row, col, radii(rad), cosa, sina,
xducerOffset, phiAngles, 0 );
                %xcoor = round(col + cosrad(rad,:));
                %ycoor = round(row + sinrad(rad,:));
```

47

```
            for i = 1:len
                if ( prows(i) > 0 & prows(i) <= nrow & pcols(i) > 0 & pcols(i) <= ncol )
                    curr_out = out( prows(i), pcols(i), rad );
                    %out( prows(i), pcols(i), rad ) = curr_out + 1;

                    %if it is the front wall, then the center should be below it - on the lower
half of the circle
                    if ( curr_edg == 1 & prows(i) > row )
                        out( prows(i), pcols(i), rad ) = curr_out + 1;
                    end

                    %if it is the back wall, then the center should be above it - on the upper
half of the circle
                    if ( curr_edg == 2 & prows(i) < row )
                        out( prows(i), pcols(i), rad ) = curr_out + 1;
                    end
                end %if
            end % for i
        end % for rad
    end % if curr_edg
  end %for col
end %for row


function [prows, pcols] = draw_polar_circle( pcent_row, pcent_col, rad, costheta,
sintheta, xducerOffset, phiAngles, draw )
    %draw a circle in polar coordiantes
    % given a center row and column in polar coordinates, a radius in
    % cartesian, and cosine and sines of theta angles
    % yoff is the transducer offset
    % phiAngles is the list of phiAngles

    if ( draw )
        x1 = rad * costheta + ( pcent_row + xducerOffset ) * sin( phiAngles( pcent_col ) );
        y1 = rad * sintheta + ( pcent_row + xducerOffset ) * cos( phiAngles( pcent_col ) );
    else
        x1 = -rad * costheta + ( pcent_row + xducerOffset ) * sin( phiAngles( pcent_col ) );
        y1 = -rad * sintheta + ( pcent_row + xducerOffset ) * cos( phiAngles( pcent_col ) );
    end


    %now convert to polar
    rads = sqrt( x1.^2 + y1 .^2 );
    phis = atan2( x1, y1 );

    prows = round(rads - xducerOffset );
    pcols = interp1( phiAngles, 1:length(phiAngles), phis, 'nearest' );
```

## FILL CIRCLE REGION - 746

```
function out_image = fill_polar_circle( pcent_row, pcent_col, rad, xducerOffset,
phiAngles, nrow, ncol )
% fill the inside of a cirlce in polar coordiantes
% given a center row and column in polar coordinates, a radius in
% cartesian, and cosine and sines of theta angles
% yoff is the transducer offset
% phiAngles is the list of phiAngles


out_image = zeros( nrow, ncol );
sinc = sin( phiAngles( pcent_col ) );
cosc = cos( phiAngles( pcent_col ) );
rad2 = rad^2;
lhs = 0;


for x = 1:nrow
   for y = 1:(ncol-1)
      lhs = x^2 + pcent_row^2 - 2 * x * pcent_row * ( sin(phiAngles(y))*sinc +
cos(phiAngles(y))*cosc );
      if ( lhs <= rad2 )
         out_image( x, y ) = 1;
      end
   end
end
```

**Claims**

1. A system for determining amniotic fluid volume, the system comprising:

   a transceiver (10) configured to deliver radio frequency ultrasound pulses to amniotic fluid regions of a patient, to receive echoes of the pulses reflected from the amniotic fluid regions, and to convert the echoes to digital form; a computer system (52) in communication with the transceiver (10), the computer system having a microprocessor and a memory, **characterized in that** the memory further containes stored programming instructions operable by the microprocessor to associate the plurality of scanplanes (210) into a rotational array (240); and the memory further containes instructions operable by the microprocessor to determine the presence of an amniotic fluid region in each scanplane (210) and determine the amniotic fluid volume spanning between and through each scanplane (210) of the rotational array (240).

2. The system according to Claim 1, wherein a method for determining areas or volumes of structures from an image is used, the method comprising:

   acquiring (410) a digital image;
   enhancing (418) the digital image using non-linear filters;
   segmenting the digital image using an intensity-based first segmentation method or algorithm (422), an edge-based second segmentation method or algorithm (438), and a combining step or algorithm (422) that combines the first and the second methods to produce homogeneous regions; and
   determining the areas of the homogenous regions.

3. The system according to Claim 2, the method determining volumes of structures from a set of images, the method comprising:

    acquiring (410) at least two digital images;
    enhancing (418) each digital image using non-linear filters;
    segmenting each digital image using an intensity-based segmentation method (422), an edge-based segmentation method (438), and a combining step (422) that combines the intensity-based and the edge-based methods to produce homogeneous regions in each digital image; and
    determining the volumes of the homogenous regions.

4. The system according to Claim 2 or 3, wherein the intensity-based segmentation method (422) includes:

    categorizing (522) the pixels into white, gray, and black pixel intensity clusters;
    assigning each pixel of each intensity cluster to a k-cluster;
    defining a cluster center by calculating a mean intensity for each pixel in the k- cluster; and
    determining a boundary by calculating the mid points between cluster centers.

5. The system according to Claim 2, 3, or 4,
wherein the edge-base segmentation method (438) includes a spatial gradients step (526), a hysteresis threshold step (530), a region-of-interest selection step (534), and a matching edges filter step (538).

6. The system according to any of Claims 2-5,
wherein the matching edges filter step includes determining the presence of boundary edge locations along a scanline by establishing that an ascending pixel gradient is followed by or paired with a decending pixel gradient.

7. The system according to any of Claims 2-6,
wherein the matching edges filter step includes determining the presence of boundary edge locations along a scanline by establishing that a descending pixel gradient is followed by or paired with an ascending pixel gradient.

8. The system according to any of Claims 2-7,
wherein the digital image is obtained from a plurality of imaging sources including computerized tomography, ultrasound imaging, magnetic resonance imaging, x-ray imaging, visible light imaging, ultraviolet imaging, microwave imaging, gamma ray imaging, infra-red imaging, positron imaging, and facsimile transmissions.

9. The system of any of Claims 2-8, wherein the matching edges filter steps includes determining the absence of boundary edge locations along a scanline by establishing that an ascending pixel gradient is unpaired with a descending pixel gradient, or that a decending pixel gradient is unpaired with an ascending gradient.

10. The system according to any of claims 2-9,
wherein the first method is an intensity-based segmentation method (422) and the second method is an edge-based segmentation method (438).

11. The system according to any of claims 2-10, wherein:

    the first algorithm is a spatial gradient algorithm, the second algorithm a initial point positioning algorithm, an optimal path algorithm that combining the spatial gradient and the initial point positioning algorithms to produce homogeneous regions.

12. The system of any of Claims 2-11, wherein the non-linear filters include a heat filter (514) and a shock filter (518), the heat filter (514) applied to the digital image followed by application of the shock filter (518) to the digital image.

13. The system of Claim 12, wherein the heat filter (514) is defined by a partial differential equation of an inputted image pixel intensity u expressed in an equation defined as

$$\frac{\partial u}{\partial t} = \frac{\partial^2 u}{\partial x^2} + \frac{\partial^2 u}{\partial y^2},$$

where u is the image being processed, $\dfrac{\partial^2 u}{\partial x^2}$ is the second partial derivative of u along the x-axis, and $\dfrac{\partial^2 u}{\partial y^2}$ is the second partial derivative of u along the y-axis.

14. The system of Claim 12 or 13, wherein the shock filter (518) is a partial differential equation of an imputed image pixel intensity u expressed in an equation defined as

$$\frac{\partial u}{\partial t} = -sign(\frac{\partial^2 u}{dx^2})\left|\frac{\partial u}{dx}\right| \,,$$

where u is the image being processed, $\dfrac{\partial^2 u}{\partial x^2}$ is the second partial derivative of u, $\left|\dfrac{\partial u}{dx}\right|$ is the absolute value of the first derivative of u along the x-axis.

15. The system of Claim 12, 13 or 14, wherein the optimum path algorithm includes a total cost function $C(p)$ weighted between an edge distance cost function, a path direction cost function, and a previous contour distance cost function from the equation

$$C(p) = \alpha C_e(p) + \beta C_d(p) + \gamma C_c(p) \,,$$

where $C_e(p)$ is the edge distance cost function, $C_d(p)$ is the path direction cost function, $C_c(p)$ is the previous contour distance cost function, and $\alpha, \beta,$ and $\gamma$ are numerical values.

16. The system of Claim 15, wherein the edge distance function $C_e(p)$ is defined from the equation

$$C_e(p) = \sum_{i=p1}^{p2} (\frac{1}{\mu + \|\nabla I_i\|}) \,,$$

where $I_i$ is the pixel intensity, $\|\nabla I_i\|$ is the gradient magnitude the pixel at location i, and $\mu$ is a constant value.

17. The system of Claim 15 or 16, wherein the path direction cost function $C_d(p)$ is defined from the equation

$$C_d(p) = \sum_{i=p1}^{p2} (I_i^{in} - I_i^{out}) \,,$$

where $I_i^{in}$ is the image intensity of the inside and dark pixel and $I_i^{out}$ is the image intensities the outside and bright pixel along a path bisecting the $I_i^{in}$ and $I_i^{out}$ pixels and connecting pixels points $i$ and $i_{-1}$ adjacent to each side of the $I_i^{in}$ pixel.

18. The system of Claim 15, 16 or 17, wherein the previous contour function $C_c(p)$ is defined by the equation

$$C_c(p) = \sum_{i=p1}^{p2} D_i^P \,,$$

where $P$ is the previous contour and $D_i^P$ is the distance of point i from the closest point on the previous contour.

19. The system according to any of claims 2-18, comprising:

   assembling the digital images into an array (240).

20. The system of claim 19, wherein the array (240) includes a rotational assembly, a wedge assembly, and a translational assembly.

21. The system according to claim 1, wherein a method to determine volume of a structure in digital images acquired from electromagnetic and non-electromagnetic sources is used, the method comprising:

   positioning a transceiver (10) exterior to a patient such that at least a portion of the structure is within a field of view of the transceiver (10), the transceiver (10) configured to send the radiation and to receive echoes of the radiation;
   sending the radiation from the transceiver to the structure;
   receiving echoes of the radiation reflected from the structure to the transceiver;
   associating the received echoes to form a plurality of 2D scanplanes (210) so that they form an array (240);
   enhancing the images of the structure in each plane (210) of the array (240) using non-linear filters and a plurality of algorithms; and
   determining the structure volume spanning between and through each plane (210) in the array (240).

22. The system of Claim 21, wherein plurality of 2D scanplanes (210) are assembled into a plurality of arrays (240) including a rotational array (240), a translational array, or a wedge array.

23. The system according to Claim 1, wherein a method to determine amniotic fluid volume in digital images is used, the method comprising:

   positioning an ultrasound transceiver (10) exterior to a patient such that at least a portion of the amniotic fluid is within a field of view of the transceiver (10), the ultrasound transceiver (10) configured to send radio frequency ultrasound pulses and to receive echoes of the radio frequency ultrasound pulses;
   sending the radio frequency ultrasound pulses from the ultrasound transceiver to amniotic fluid regions;
   receiving echoes of the radio frequency ultrasound pulses reflected from the amniotic fluid regions to the transceiver (10);
   associating the received echoes to form a plurality of 2D scanplanes (210) so that they form an array (240);
   enhancing the images of the amniotic fluid regions in each plane (210) of the array (240) using a plurality of algorithms; and
   determining the amniotic fluid volume of the amniotic fluid regions spanning between and through each plane (210) in the array (240).

24. The system of Claim 23, wherein plurality of 2D scanplanes (210) are acquired from a rotational array (240), a translational array, or a wedge array.

25. The system of Claim 23 or 24, wherein the plurality of 2D scanplanes (210) includes at least two scanplanes.

26. The system of Claim 23, 24 or 25, wherein the radio frequency ultrasound is within a range from approximately 2 MHz to approximately 10 MHz.

27. The system of any of Claims 23-26, wherein the plurality of algorithms includes algorithms for image enhancement, segmentation, and polishing.

28. The system of Claim 27, wherein segmentation further includes an intensity clustering step (522), a spatial gradients step (526), a hysteresis threshold step (530), a Region-of-Interest selection step (534), and a matching edges filter step (538).

29. The system of Claim 28, wherein the intensity clustering step (522) is performed in a first parallel operation, and the spatial gradients (526), hysteresis threshold (530), Region-of-Interest selection (534), and matching edges filter steps (538) are performed in a second parallel operation, and further wherein the results from the first parallel operation are combined with the results from the second parallel operation.

30. The system of any of Claims 22-29, using a method as comprised by the systems according to any of Claims 2-22.

31. The system of any of claims 22-30, wherein the amniotic fluid volume is adjusted for underestimation or overestimation.

32. The system of Claim 31, wherein the amniotic fluid volume is adjusted for underestimation by probing with adjustable ultrasound frequencies to penetrate deep tissues and to repositioning the transceiver (10) to establish that deep tissues are exposed with probing ultrasound of sufficient strength to provide a reflecting ultrasound echo receivable by the transceiver (10), such that more than one rotational array (240) to detect deep tissue and regions of the fetal head are obtained.

33. The system of Claim 31 or 32, wherein amniotic fluid volume is adjusted for overestimation by automatically determining fetal head volume contribution to amniotic fluid volume and deducting it from the amniotic fluid volume.

34. The system of Claim 33, wherein the steps to adjust for overestimated amniotic fluid volumes include a 2D clustering step, a matching edges step, an all edges step, a gestational age factor step (726), a head diameter step (730), an head edge detection step (734), and a Hough transform step (736).

35. The system of Claim 34, wherein the Hough transform step includes a polar Hough Transform step (738), a Find Maximum Hough value step (742), and a fill circle region step (746).

36. The system of Claim 35, wherein the polar Hough Transform step includes a first Hough transform to look for lines of a specified shape, and a second Hough transform to look for fetal head structures.

37. The system of any of Claims 1-36, wherein each scanplane (210) is arranged as a plurality of scanlines (214), each scanline (214) of the plurality of scanlines being separated by approximately 1.5 degrees and having a length suitable for the dimension of amniotic fluid region.

38. The system of any of Claims 1-37, wherein each scanplane (210) in the plurality of scanplanes (210) is separated from an adjacent scanplane (210) in the plurality of scanplanes (210) by approximately 7.5 degrees.

39. The system of any of Claims 1-38, wherein the transceiver (10) includes a display (24) to present the graphic image of a scanplane (210) in two-dimensions and the rotational array (240) in three-dimensions.

40. The system of any of Claims 1-39, wherein the computer system (52) is configured for remote operation via an Internet web-based system, the internet web-based system having a plurality of programs that collect, analyze, and store amniotic fluid volume.


**Patentansprüche**

1. System zum Bestimmen eines amniotischen Fluidvolumens, wobei das System umfasst:

einen Sendeempfänger (10), der ausgelegt ist, Funkfrequenz-Ultraschallpulse an amniotische Fluidbereiche eines Patienten abzugeben, um Echos der von den amniotischen Fluidbereichen reflektierten Pulse zu empfangen und die Echos in digitale Form zu konvertieren;
ein Computersystem (52) in Verbindung mit dem Sendeempfänger (10), wobei das Computersystem einen Mikroprozessor und einen Speicher aufweist, **dadurch gekennzeichnet, dass** der Speicher ferner gespeicherte Programmierinstruktionen umfasst, die vom Mikroprozessor ausführbar sind, um die Vielzahl von Scanebenen (210) in ein Rotationsarray (240) zu vereinigen; und
der Speicher ferner Instruktionen enthält, die vom Mikroprozessor durchführbar sind, um die Anwesenheit amniotischer Fluidbereiche in jeder Scanebene (210) zu bestimmen und das amniotische Fluidvolumen zu bestimmen, das sich zwischen und durch jede Scanebene (210) des Rotationsarrays (240) spannt.

2. System gemäß Anspruch 1, wobei ein Verfahren zum Bestimmen von Flächen oder Volumen von Strukturen aus einem Bild verwendet wird, wobei das Verfahren umfasst:

Erfassen (410) eines digitalen Bildes;
Verstärken (418) des digitalen Bildes unter Verwendung nicht-linearer Filter;
Segmentieren des digitalen Bildes unter Verwendung eines intensitätsbasierten ersten Segmentationsverfahrens oder -algorithmus (422), eines kantenbasierten zweiten Segmentationsverfahrens oder -algorithmus (438) und eines Kombinierungsschritts oder -algorithmus (422), der das erste und das zweite Verfahren kombiniert, um homogene Bereiche zu erzeugen; und
Bestimmen der Flächen der homogenen Bereiche.

3. System gemäß Anspruch 2, wobei das Verfahren Volumen von Strukturen eines Satzes von Bildern bestimmt, wobei das Verfahren umfasst:

Erfassen (410) wenigstens zweier digitaler Bilder;
Verstärken (418) jedes digitalen Bildes unter Verwendung nicht-linearer Filter;
Segmentieren jedes digitalen Bildes unter Verwendung eines intensitätsbasierten Segmentationsverfahrens (422), eines kantenbasierten Segmentationsverfahrens (438) und eines Kombinierungsschritts (422), der das intensitätsbasierte und das kantenbasierte Verfahren kombiniert, um homogene Bereiche in jedem digitalen Bild zu erzeugen; und
Bestimmen der Volumina der homogenen Bereiche.

4. System gemäß Anspruch 2 oder 3, wobei das intensitätsbasierte Segmentationsverfahren (422) umfasst:

Kategorisieren (522) der Pixel in Intensitätscluster mit weißen, grauen und schwarzen Pixeln;
Zuweisen jedes Pixels jedes Intensitätsclusters an ein k-Cluster;
Festlegen eines Clusterzentrums durch Berechnen einer mittleren Intensität für jedes Pixel im k-Cluster; und
Bestimmen einer Grenze durch Berechnen der Mittelpunkte zwischen Clusterzentren.

5. System gemäß Anspruch 2, 3 oder 4, wobei das kantenbasierte Segmentationsverfahren (438) einen räumlichen Gradientenschritt (526), einen Hystereseschwellwertschritt (530), einen Bereich-von-Interesse-Auswahlschritt (534) und einen Übereinstimmende-Kanten-Filterschritt (538).

6. System gemäß einem der Ansprüche 2-5, wobei der Übereinstimmende-Kanten-Filterschritt ein Bestimmen der Anwesenheit von Grenzkantenorten entlang einer Scanlinie erfasst durch Ermitteln, dass ein ansteigender Pixelgradient gefolgt ist von oder gepaart ist mit einem absteigenden Pixelgradienten.

7. System gemäß einem der Ansprüche 2-6, wobei der Übereinstimmende-Kanten-Filterschritt ein Bestimmen der Anwesenheit von Grenzkantenorten entlang einer Scanlinie erfasst durch Ermitteln, dass ein absteigender Pixelgradient gefolgt ist von oder gepaart ist mit einem ansteigenden Pixelgradienten.

8. System gemäß einem der Ansprüche 2-7, wobei das digitale Bild aus einer Vielzahl von bildgebenden Quellen erhalten wird, die Computertomografie, Ultraschallbildgebung, Kernspintomografie, Röntgenstrahlen-Bildgebung, Bildgebung durch sichtbares Licht, Ultraviolett-Bildgebung, Mikrowellen-Bildgebung, Gammastrahlen-Bildgebung, Infrarot-Bildgebung, Positron-Bildgebung und Faxübertragungen.

9. System gemäß einem der Ansprüche 2-8, wobei die Übereinstimmende-Kanten-Filterschritte ein Bestimmen der Abwesenheit von Grenzkantenorten entlang einer Scanlinie umfassen durch Ermitteln, dass ein aufsteigender Pixelgradient nicht mit einem absteigenden Pixelgradienten gepaart ist, oder dass ein absteigender Pixelgradient nicht mit einem ansteigenden Gradienten gepaart ist.

10. System gemäß einem der Ansprüche 2-9, wobei das erste Verfahren ein intensitätsbasiertes Segmentationsverfahren (422) ist und das zweite Verfahren ein kantenbasiertes Segmentationsverfahren (438) ist.

11. System gemäß einem der Ansprüche 2-10, wobei:

der erste Algorithmus ein räumlicher Gradientenalgorithmus ist, der zweite Algorithmus ein Anfangspunktpositionierungsalgorithmus ist, wobei ein optimaler Pfadalgorithmus den räumlichen Gradientenalgorithmus und den Anfangspunktpositionierungsalgorithmus kombiniert, um homogene Bereiche zu erzeugen.

**12.** System gemäß einem der Ansprüche 2-11, wobei die nicht-linearen Filter einen Wärmefilter (514) und einen Schockfilter (518) umfassen, wobei der Wärmefilter (514) auf das digitale Bild angewendet wird gefolgt von einer Anwendung des Schockfilters (518) auf das digitale Bild.

**13.** System gemäß Anspruch 12, wobei der Wärmefilter (514) festgelegt ist durch eine partielle Differentialgleichung einer eingegebenen Bildpixelintensität u, die in einer Gleichung ausgedrückt ist, die definiert ist als

$$\frac{\partial u}{\partial t} = \frac{\partial^2 u}{\partial x^2} + \frac{\partial^2 u}{\partial y^2},$$

wobei u Bild ist, das verarbeitet wird, $\frac{\partial^2 u}{\partial x^2}$ die zweite partielle Ableitung von u entlang der x-Achse ist, und $\frac{\partial^2 u}{\partial y^2}$ die zweite partielle Ableitung von u entlang der y-Achse ist.

**14.** System gemäß Anspruch 12 oder 13, wobei der Schockfilter (518) eine partielle Differentialgleichung einer eingegebenen Bildpixelintensität u ist, die in einer Gleichung ausgedrückt ist, die definiert ist als

$$\frac{\partial u}{\partial t} = -sign(\frac{\partial^2 u}{\partial x^2}) \mid \frac{\partial u}{\partial x} \mid,$$

wobei u Bild ist, das verarbeitetet wird, $\frac{\partial^2 u}{\partial x^2}$ die zweite partielle Ableitung von u entlang der x-Achse ist, und $\mid \frac{\partial u}{\partial x} \mid$ der absolute Wert der ersten Ableitung von u entlang der x-Achse ist.

**15.** System gemäß Anspruch 12, 13 oder 14, wobei der optimale Pfadalgorithmus eine Gesamtkostenfunktion C(p) umfasst, die gewichtet ist zwischen einer Kantenabstandskostenfunktion, einer Pfadrichtungsfunktion und einer früheren Konturabstandskostenfunktion aus der Gleichung

$$C(p) = \alpha C_e(p) + \beta C_d(p) + \gamma C_c(p),$$

wobei $C_e$(p) die Kantenabstandskostenfunktion ist, $C_d$(p) die Pfadrichtungskostenfunktion ist, $C_c$(p) die vorherige Konturabstandskostenfunktion ist, und $\alpha$, $\beta$ und $\gamma$ numerische Werte sind.

**16.** System gemäß Anspruch 15, wobei die Kantenabstandsfunktion $C_e$(p) definiert ist aus der Gleichung

$$C_e(p) = \sum_{i=p1}^{p2} (\frac{1}{\mu + \|\nabla I_i\|}),$$

wobei $I_i$ die Pixelintensität ist, $\|\nabla I_i\|$ die Gradientengröße des Pixels am Ort i ist und $\mu$ ein konstanter Wert ist.

**17.** System gemäß Anspruch 15 oder 16, wobei die Pfadrichtungskostenfunktion $C_d$(p) definiert ist aus der Gleichung

$$C_d(p) = \sum_{i=p1}^{p2} (I_i^{in} - I_i^{out}),$$

wobei $I_i^{in}$ die Bildintensität der Innenseite und dunkle Pixel ist und $I_i^{out}$ die Bildintensitäten der Außenseite und helle Pixel entlang eines Pfades ist, der die $I_i^{in}$ und $I_i^{out}$ Pixel zweiteilt und Pixelpunkte i und $i_{-1}$ benachbart zu jeder Seite des $I_i^{in}$ Pixels verbindet.

**18.** System gemäß Anspruch 15, 16 oder 17, wobei die vorherige Konturfunktion C$_c$(p) definiert ist durch die Gleichung

$$C_c(p) = \sum_{i=p1}^{p2} D_i^P \ ,$$

wobei P die vorherige Kontur ist und $D_i^P$ der Abstand des Punktes i vom nächstliegenden Punkt auf der vorherigen Kontur ist.

**19.** System gemäß einem der Ansprüche 2-18, umfassend:

Zusammenstellen der digitalen Bilder in ein Feld (240).

**20.** System gemäß Anspruch 19, wobei das Array (240) eine Rotationsanordnung, eine Keilanordnung und eine Translationsanordnung umfasst.

**21.** System gemäß Anspruch 1, wobei ein Verfahren zum Bestimmen von Volumen einer Struktur in digitalen Bildern, die von elektromagnetischen und nichtelektromagnetischen Quellen aufgenommen wurden, verwendet wird, wobei das Verfahren umfasst:

Positionieren eines Sendeempfängers (10) außerhalb eines Patienten, so dass wenigstens ein Teil der Struktur innerhalb eines Sichtfelds des Sendeempfängers (10) ist, wobei der Sendeempfänger (10) ausgelegt ist, die Strahlung zu senden und Echos der Strahlung zu empfangen;
Senden der Strahlung vom Sendeempfänger zur Struktur;
Empfangen von Echos der von der Struktur zum Sendeempfänger reflektierten Strahlung;
Zuweisen der empfangenen Echos, um eine Vielzahl von 2D-Scanebenen (210) zu bilden, so dass sie ein Array (240) bilden;
Verstärken der Bilder der Struktur in jeder Ebene (210) des Arrays (240) unter Verwendung nicht-linearer Filter und einer Vielzahl von Algorithmen; und
Bestimmen des Strukturvolumens, das sich zwischen und durch jede Ebene (210) in dem Array (240) spannt.

**22.** System gemäß Anspruch 21, wobei eine Vielzahl von 2D-Scanebenen (210) in eine Vielzahl von Arrays (240) zusammengefügt sind, die ein Rotationsarray (240), ein Translationsarray oder ein Keilarray umfassen.

**23.** System gemäß Anspruch 1, wobei ein Verfahren zum Bestimmen eines amniotischen Fluidvolumens in digitalen Bildern verwendet wird, wobei das Verfahren umfasst:

Positionieren eines Ultraschall-Sendeempfängers (10) außerhalb eines Patienten, so dass wenigstens ein Teil des amniotischen Fluids innerhalb eines Sichtfelds des Sendeempfängers (10) ist, wobei der Ultraschall-Sendeempfänger (10) ausgelegt ist, um Funkfrequenz-Ultraschallpulse zu senden und Echos der Funkfrequenz-Ultraschallpulse zu empfangen;
Senden der Funkfrequenz-Ultraschallpulse vom Ultraschall-Sendeempfänger zu amniotischen Fluidbereichen;
Empfangen von Echos der von den amniotischen Fluidbereichen reflektierten Funkfrequenz-Ultraschallpulse zum Sendeempfänger (10);
Zuordnen der empfangenen Echos, um eine Vielzahl von 2D-Scanebenen (210) zu bilden, so dass sie ein Array (240) bilden;
Verstärken der Bilder der amniotischen Fluidbereiche in jeder Ebene (210) des Arrays (240) unter Verwendung einer Vielzahl von Algorithmen; und
Bestimmen des amniotischen Fluidvolumens der amniotischen Fluidbereiche, die sich zwischen und durch jede Ebene (210) in dem Array (240) spannen.

24. System gemäß Anspruch 23, wobei eine Vielzahl von 2D-Scanebenen (210) von einem Rotationsarray (240), einem Translationsarray oder einem Keilarray erfasst werden.

25. System gemäß Anspruch 23 oder 24, wobei die Vielzahl von 2D-Scanebenen (210) wenigstens zwei Scanebenen umfassen.

26. System gemäß Anspruch 23, 24 oder 25, wobei der Funkfrequenz-Ultraschall innerhalb eines Bereichs von ungefähr 2 MHz bis ungefähr 10 MHz ist.

27. System gemäß einem der Ansprüche 23-26, wobei die Vielzahl von Algorithmen Algorithmen zur Bildverstärkung, -segmentierung und -polierung umfassen.

28. System gemäß Anspruch 27, wobei Segmentierung ferner einen Intensitätsclusterungsschritt (522), einen Raumgradientenschritt (526), einen Hystereseschwellwertschritt (530), einen Bereich-von-Interesse-Auswahlschritt (534) und einen Übereinstimmende-Kanten-Filterschritt (538) umfasst.

29. System gemäß Anspruch 28, wobei der Intensitätsclusterungsschritt (522) in einer ersten parallelen Operation durchgeführt wird, und die räumlichen Gradienten (526), Hystereseschwellwert (530), Bereich-von-Interesse-Auswahl (534) und Übereinstimmende-Kanten-Filterschritte (538) in einer zweiten parallelen Operation durchgeführt werden, und wobei ferner die Ergebnisse aus der ersten parallelen Operation mit den Ergebnissen aus der zweiten parallelen Operation kombiniert werden.

30. System gemäß einem der Ansprüche 22-29, welches ein Verfahren, wie durch die Systeme gemäß einem der Ansprüche 2-22 umfasst, verwendet.

31. System gemäß einem der Ansprüche 22-30, wobei das amniotische Fluidvolumen zur Unterschätzung oder Überschätzung angepasst wird.

32. System gemäß Anspruch 31, wobei das amniotische Fluidvolumen zur Unterschätzung angepasst wird durch Prüfen mit anpassbaren Ultraschallfrequenzen, um tiefe Gewebe zu durchdringen und um den Sendeempfänger (10) neu zu positionieren, um zu ermitteln, dass tiefe Gewebe einem Prüfultraschall ausreichender Stärke ausgesetzt sind, um ein reflektierendes Ultraschallecho bereitzustellen, das von dem Sendeempfänger (10) empfangbar ist, so dass mehr als ein Rotationsarray (240), um tiefes Gewebe und Bereiche des fötalen Kopfes zu detektieren, erhalten werden.

33. System gemäß Anspruch 31 oder 32, wobei amniotisches Fluidvolumen angepasst wird zur Überschätzung durch automatisches Bestimmen des Beitrags des fötalen Kopfvolumens zum amniotischen Fluidvolumen und um es vom amniotischen Fluidvolumen abzuziehen.

34. System gemäß Anspruch 33, wobei die Schritte zur Anpassung an überschätzte amniotische Fluidvolumen einen 2D-Clusterschritt, einen Übereinstimmende-Kanten-Schritt, einen Alle-Kanten-Schritt, einen Schwangerschaftsalterfaktorschritt (726), einen Kopfdurchmesserschritt (730), einen Kopfkantendetektierungsschritt (734) und einen Houghtransformationsschritt umfassen.

35. System gemäß Anspruch 34, wobei der Houghtransformationsschritt einen Polare-Houghtransformationsschritt (738), einen Finde-Maximum-Houghwert-Schritt (742) und einen Fülle-Kreisbereich-Schritt (746).

36. System gemäß Anspruch 35, wobei der Polare-Houghtransformationsschritt eine erste Houghtransformation umfasst, um nach Linien einer speziellen Form zu suchen, und eine zweite Houghtransformation, um nach fötalen Kopfstrukturen zu suchen.

37. System gemäß einem der Ansprüche 1-36, wobei jede Scanebene (210) als eine Vielzahl von Scanlinien (214) ausgelegt ist, wobei jede Scanlinie (214) der Vielzahl von Scanlinien durch ungefähr 1.5 Grad getrennt ist und ein Länge aufweist, die für die Abmessung eines amniotischen Fluidbereichs geeignet ist.

38. System gemäß einem der Ansprüche 1-37, wobei jede Scanebene (210) in der Vielzahl von Scanebenen (210) von einer benachbarten Scanebene (210) in der Vielzahl von Scanebenen (210) um ungefähr 7.5 Grad getrennt ist.

**39.** System gemäß einem der Ansprüche 1-38, wobei der Sendeempfänger (10) eine Anzeige (24) umfasst, um das graphische Bild einer Scanebene (210) in zwei Dimensionen und das Rotationsarray (240) in drei Dimensionen darzustellen.

**40.** System gemäß einem der Ansprüche 1-39, wobei das Computersystem (52) zum entfernten Betrieb über ein auf Internet und Web basiertes System ausgelegt ist, wobei das auf Internet und Web basierte System eine Vielzahl von Programmen aufweisen, die ein amniotisches Fluidvolumen sammeln, analysieren und speichern.

**Revendications**

**1.** Système pour déterminer un volume de liquide amniotique, le système comprenant :

un émetteur-récepteur (10) configuré pour délivrer des impulsions ultrasonores radiofréquence à des régions de liquide amniotique d'un patient, pour recevoir des échos des impulsions réfléchies depuis les régions de liquide amniotique, et pour convertir les échos sous forme numérique ;
un système informatique (52) en communication avec l'émetteur-récepteur (10), le système informatique ayant un microprocesseur et une mémoire, **caractérisé en ce que** la mémoire contient en outre des instructions de programmation stockées exploitables par le microprocesseur pour associer la pluralité de plans de balayage (210) dans une matrice de rotation (240) ; et
la mémoire contient en outre des instructions exploitables par le microprocesseur pour déterminer la présence d'une région de liquide amniotique dans chaque plan de balayage (210) et déterminer le volume de liquide amniotique s'étendant entre et à travers chaque plan de balayage (210) de la matrice de rotation (240).

**2.** Système selon la revendication 1, dans lequel un procédé pour déterminer des aires ou des volumes de structures à partir d'une image est utilisé, le procédé comprenant :

l'acquisition (410) d'une image numérique ;
l'amélioration (418) de l'image numérique en utilisant des filtres non linéaires ;
la segmentation de l'image numérique en utilisant un premier procédé ou algorithme de segmentation basé sur l'intensité (422), un second procédé ou algorithme de segmentation basé sur les bords (438), et une étape ou un algorithme de combinaison (422) qui combine le premier et le second procédé afin de produire des régions homogènes ; et
la détermination des aires des régions homogènes.

**3.** Système selon la revendication 2, le procédé déterminant des volumes de structures à partir d'un ensemble d'images, le procédé comprenant :

l'acquisition (410) d'au moins deux images numériques ;
l'amélioration (418) de chaque image numérique en utilisant des filtres non linéaires ;
la segmentation de chaque image numérique en utilisant un procédé de segmentation basé sur l'intensité (422), un procédé de segmentation basé sur les bords (438), et une étape de combinaison (422) qui combine le procédé basé sur l'intensité et le procédé basé sur les bords afin de produire des régions homogènes dans chaque image numérique ; et
la détermination des volumes des régions homogènes.

**4.** Système selon la revendication 2 ou 3, dans lequel le procédé de segmentation basé sur l'intensité (422) inclut :

la catégorisation (522) des pixels en grappes d'intensité de pixels blanc, gris et noir ;
l'affectation de chaque pixel de chaque grappe d'intensité à une grappe k ;
la définition d'un centre de grappe en calculant une intensité moyenne pour chaque pixel dans la grappe k ; et
la détermination d'une limite en calculant les points médians entre des centres de grappes.

**5.** Système selon la revendication 2, 3 ou 4, dans lequel le procédé de segmentation basé sur les bords (438) inclut une étape de gradients spatiaux (526), une étape de seuil d'hystérésis (530), une étape de sélection de région d'intérêt (534), et une étape de filtre de bords concordants (538).

**6.** Système selon l'une quelconque des revendications 2 à 5, dans lequel l'étape de filtre de bords concordants inclut la détermination de la présence d'emplacements de bord limite le long d'une ligne de balayage en établissant qu'un gradient de pixel ascendant est suivi de ou apparié avec un gradient de pixel descendant.

**7.** Système selon l'une quelconque des revendications 2 à 6, dans lequel l'étape de filtre de bords concordants inclut la détermination de la présence d'emplacements de bord limite le long d'une ligne de balayage en établissant qu'un gradient de pixel descendant est suivi de ou apparié avec un gradient de pixel ascendant.

**8.** Système selon l'une quelconque des revendications 2 à 7, dans lequel l'image numérique est obtenue à partir d'une pluralité de sources d'imagerie incluant la tomodensitométrie, l'imagerie ultrasonore, l'imagerie par résonance magnétique, l'imagerie par rayons X, l'imagerie par lumière visible, l'imagerie ultraviolette, l'imagerie par micro-ondes, l'imagerie par rayons gamma, l'imagerie infrarouge, l'imagerie à positons, et les émissions de télécopie.

**9.** Système selon l'une quelconque des revendications 2 à 8, dans lequel l'étape de filtre de bords concordants inclut la détermination de l'absence d'emplacements de bord limite le long d'une ligne de balayage en établissant qu'un gradient de pixel ascendant n'est pas apparié avec un gradient de pixel descendant, ou qu'un gradient de pixel descendant n'est pas apparié avec un gradient de pixel ascendant.

**10.** Système selon l'une quelconque des revendications 2 à 9, dans lequel le premier procédé est un procédé de segmentation basé sur l'intensité (422) et le second procédé est un procédé de segmentation basé sur les bords (438).

**11.** Système selon l'une quelconque des revendications 2 à 10, dans lequel :

le premier algorithme est un algorithme de gradient spatial, le second algorithme est un algorithme de positionnement de point initial, un algorithme de chemin optimal qui combine l'algorithme de gradient spatial et l'algorithme de positionnement de point initial afin de produire des régions homogènes.

**12.** Système selon l'une quelconque des revendications 2 à 11, dans lequel les filtres non linéaires incluent un filtre anticalorifique (514) et un filtre de choc (518), le filtre anticalorifique (514) appliqué à l'image numérique suivi de l'application du filtre de choc (518) à l'image numérique.

**13.** Système selon la revendication 12, dans lequel le filtre anticalorifique (514) est défini par une équation différentielle partielle d'une intensité de pixel d'image imputée u exprimée dans une équation définie comme

$$\frac{\partial u}{\partial t} = \frac{\partial^2 u}{\partial x^2} + \frac{\partial^2 u}{\partial y^2},$$

où u est l'image qui est traitée, $\frac{\partial^2 u}{\partial x^2}$ est la seconde dérivée partielle de u le long de l'axe x, et $\frac{\partial^2 u}{\partial y^2}$ est la seconde dérivée partielle de u le long de l'axe y.

**14.** Système selon la revendication 12 ou 13, dans lequel le filtre de choc (518) est une équation différentielle partielle d'une intensité de pixel d'image imputée u exprimée dans une équation définie comme

$$\frac{\partial u}{\partial t} = signe - (\frac{\partial^2 u}{dx^2})\left|\frac{\partial u}{dx}\right|,$$

où u est l'image qui est traitée, $\frac{\partial^2 u}{\partial x^2}$ est la seconde dérivée partielle de u, et $\left|\frac{\partial u}{dx}\right|$ est la valeur absolue de la première dérivée de u le long de l'axe x.

**15.** Système selon la revendication 12, 13 ou 14, dans lequel l'algorithme de chemin optimal inclut une fonction de coût total C(p) pondérée entre une fonction de coût de distance de bord, une fonction de coût de direction de chemin, et une fonction de coût de distance de contour précédent à partir de l'équation

$$C(p) = \alpha C_e(p) + \beta C_d(p) + \gamma C_c(p),$$

où $C_e(p)$ est la fonction de coût de distance de bord, $C_d(p)$ est la fonction de coût de direction de chemin, $C_c(p)$ est la fonction de coût de distance de contour précédent, et $\alpha$, $\beta$ et $\gamma$ sont des valeurs numériques.

**16.** Système selon la revendication 15, dans lequel la fonction de distance de bord $C_e(p)$ est définie à partir de l'équation

$$C_e(p) = \sum_{i=p1}^{p2}\left(\frac{1}{\mu + \|\nabla I_i\|}\right),$$

où $I_i$ est l'intensité de pixel, $\|\nabla I_i\|$ est la grandeur de gradient du pixel à l'emplacement i, et $\mu$ est une valeur constante.

**17.** Système selon la revendication 15 ou 16, dans lequel la fonction de coût de direction de chemin $C_d(p)$ est définie à partir de l'équation

$$C_d(p) = \sum_{i=p1}^{p2}(I_i^{in} - I_i^{out}),$$

où $I_i^{in}$ est l'intensité d'image du pixel intérieur et sombre et $I_i^{out}$ est l'intensité d'image du pixel extérieur et brillant le long d'un chemin bissectant les pixels $I_i^{in}$ et $I_i^{out}$ et reliant les points de pixels $i$ et $i_{-1}$ adjacents à chaque côté du pixel $I_i^{in}$.

**18.** Système selon la revendication 15, 16 ou 17, dans lequel la fonction de contour précédent $C_c(p)$ est définie par l'équation

$$C_c(p) = \sum_{i=p1}^{p2}D_i^P,$$

où P est le contour précédent et $D_i^P$ est la distance du point i depuis le point le plus proche sur le contour précédent.

**19.** Système selon l'une quelconque des revendications 2 à 18, comprenant :

l'assemblage des images numériques en une matrice (240).

**20.** Système selon la revendication 19, dans lequel la matrice (240) inclut un assemblage de rotation, un assemblage oblique, et un assemblage de translation.

**21.** Système selon la revendication 1, dans lequel un procédé pour déterminer le volume d'une structure dans des images numériques acquises depuis des sources électromagnétiques et non électromagnétiques est utilisé, le procédé comprenant :

le positionnement d'un émetteur-récepteur (10) extérieur à un patient de sorte qu'au moins une partie de la structure soit à l'intérieur d'un champ de vue de l'émetteur-récepteur (10), l'émetteur-récepteur (10) configuré pour envoyer le rayonnement et pour recevoir des échos du rayonnement ;

l'envoi du rayonnement de l'émetteur-récepteur à la structure ;

la réception d'échos du rayonnement réfléchi depuis la structure vers l'émetteur-récepteur;

l'association des échos reçus pour former une pluralité de plans de balayage en 2D (210) de sorte qu'ils forment une matrice (240) ;

l'amélioration des images de la structure dans chaque plan (210) de la matrice (240) en utilisant des filtres non linéaires et une pluralité d'algorithmes ; et

la détermination du volume de la structure s'étendant entre et à travers chaque plan (210) dans la matrice (240).

22. Système selon la revendication 21, dans lequel une pluralité de plans de balayage en 2D (210) sont assemblés en une pluralité de matrices (240) incluant une matrice de rotation (240), une matrice de translation, ou une matrice oblique.

23. Système selon la revendication 1, dans lequel un procédé pour déterminer un volume de liquide amniotique dans des images numériques est utilisé, le procédé comprenant :

le positionnement d'un émetteur-récepteur ultrasonore (10) extérieur à un patient de sorte qu'au moins une partie du liquide amniotique soit à l'intérieur d'un champ de vue de l'émetteur-récepteur (10), l'émetteur-récepteur ultrasonore (10) configuré pour envoyer des impulsions ultrasonores radiofréquence et pour recevoir des échos des impulsions ultrasonores radiofréquence ;

l'envoi des impulsions ultrasonores radiofréquence de l'émetteur-récepteur ultrasonore à des régions de liquide amniotique ;

la réception d'échos des impulsions ultrasonores radiofréquence réfléchies depuis les régions de liquide amniotique vers l'émetteur-récepteur (10) ;

l'association des échos reçus pour former une pluralité de plans de balayage en 2D (210) de sorte qu'ils forment une matrice (240) ;

l'amélioration des images des régions de liquide amniotique dans chaque plan (210) de la matrice (240) en utilisant une pluralité d'algorithmes ; et

la détermination du volume de liquide amniotique des régions de liquide amniotique s'étendant entre et à travers chaque plan (210) dans la matrice (240).

24. Système selon la revendication 23, dans lequel une pluralité de plans de balayage en 2D (210) sont acquis à partir d'une matrice de rotation (240), d'une matrice de translation, ou d'une matrice oblique.

25. Système selon la revendication 23 ou 24, dans lequel la pluralité de plans de balayage en 2D (210) inclut au moins deux plans de balayage.

26. Système selon la revendication 23, 24 ou 25, dans lequel l'ultrason radiofréquence est à l'intérieur d'une plage allant d'approximativement 2 MHz à approximativement 10 MHz.

27. Système selon l'une quelconque des revendications 23 à 26, dans lequel la pluralité d'algorithmes inclut des algorithmes pour l'amélioration d'image, la segmentation, et le polissage.

28. Système selon la revendication 27, dans lequel la segmentation inclut en outre une étape de mise en grappe d'intensité (522), une étape de gradients spatiaux (526), une étape de seuil d'hystérésis (530), une étape de sélection de région d'intérêt (534), et une étape de filtre de bords concordants (538).

29. Système selon la revendication 28, dans lequel l'étape de mise en grappe d'intensité (522) est effectuée dans une première opération parallèle, et l'étape de gradients spatiaux (526), l'étape de seuil d'hystérésis (530), l'étape de sélection de région d'intérêt (534), et l'étape de filtre de bords concordants (538) sont effectuées dans une seconde opération parallèle, et en outre dans lequel les résultats de la première opération parallèle sont combinés avec les résultats de la seconde opération parallèle.

30. Système selon l'une quelconque des revendications 22 à 29, utilisant un procédé tel que compris par les systèmes selon l'une quelconque des revendications 2 à 22.

31. Système selon l'une quelconque des revendications 22 à 30, dans lequel le volume de liquide amniotique est ajusté pour une sous-estimation ou une surestimation.

**32.** Système selon la revendication 31, dans lequel le volume de liquide amniotique est ajusté pour une sous-estimation par sondage avec des fréquences ultrasonores ajustables pour pénétrer des tissus profonds et repositionnement de l'émetteur-récepteur (10) pour établir que des tissus profonds sont exposés avec un ultrason de sondage de force suffisante pour fournir un écho ultrasonore réfléchissant pouvant être reçu par l'émetteur-récepteur (10), de sorte que plus d'une matrice de rotation (240) pour détecter un tissu profond et des régions de la tête foetale soit obtenue.

**33.** Système selon la revendication 31 ou 32, dans lequel le volume de liquide amniotique est ajusté pour une surestimation en déterminant automatiquement la contribution du volume de la tête foetale au volume de liquide amniotique et en la déduisant du volume de liquide amniotique.

**34.** Système selon la revendication 33, dans lequel les étapes pour ajuster des volumes de liquide amniotique surestimés incluent une étape de mise en grappe en 2D, une étape de bords concordants, une étape de tous les bords, une étape de facteur d'âge gestationnel (726), une étape de diamètre de tête (730), une étape de détection de bord de tête (734), et une étape de transformée de Hough (736).

**35.** Système selon la revendication 34, dans lequel l'étape de transformée de Hough inclut une étape de transformée de Hough polaire (738), une étape consistant à trouver une valeur maximale de Hough (742), et une étape de remplissage de région de cercle (746).

**36.** Système selon la revendication 35, dans lequel l'étape de transformée de Hough polaire inclut une première transformée de Hough pour chercher des lignes d'une forme spécifiée, et une seconde transformée de Hough pour chercher des structures de tête foetale.

**37.** Système selon l'une quelconque des revendications 1 à 36, dans lequel chaque plan de balayage (210) est agencé comme une pluralité de lignes de balayage (214), chaque ligne de balayage (214) de la pluralité de lignes de balayage étant séparée d'approximativement 1,5 degré et ayant une longueur appropriée pour la dimension de la région de liquide amniotique.

**38.** Système selon l'une quelconque des revendications 1 à 37, dans lequel chaque plan de balayage (210) de la pluralité de plans de balayage (210) est séparé d'un plan de balayage (210) adjacent de la pluralité de plans de balayage (210) d'approximativement 7,5 degrés.

**39.** Système selon l'une quelconque des revendications 1 à 38, dans lequel l'émetteur-récepteur (10) inclut un afficheur (24) pour présenter l'image graphique d'un plan de balayage (210) en deux dimensions et la matrice de rotation (240) en trois dimensions.

**40.** Système selon l'une quelconque des revendications 1 à 39, dans lequel le système informatique (52) est configuré pour une opération à distance via un système Internet basé sur le Web, le système Internet basé sur le Web ayant une pluralité de programmes qui collectent, analysent et stockent un volume de liquide amniotique.

# FIG. 1

FIG. 2B

FIG. 2A

**Fig. 3**

**Fig. 4**

**FIG. 5A**

**FIG. 5B**

**FIG. 6**

Input Data
410

Image Enhancement
418

Intensity-Based
Segmentation 422

Edge-Based
Segmentation 438

Combine by AND Operator
of Images 442

Polish 464

Output Segmented
Image 480

Compute Area or
Volume 484

# FIG. 7

```
┌ ─ ─ ─ ─ ─ ─ ─ ─ ─ ─ ─ ─ ─ ┐
                          418
│  ┌──────────────────────┐  │
   │  Heat Filter    514  │
│  └──────────────────────┘  │
            │
│           ▼               │
   ┌──────────────────────┐
│  │  Shock Filter   518  │  │
   └──────────────────────┘
└ ─ ─ ─ ─ ─ ─ ─ ─ ─ ─ ─ ─ ─ ┘
```

# FIG. 8A

```
┌ ─ ─ ─ ─ ─ ─ ─ ┐
            422
│  ┌─────────┐  │
   │         │
│  │Intensity│  │
   │Clustering│
│  │into white,│ │
   │gray, and │
│  │black pixels│ │
   │         │
│  │         │  │
   │     522 │
│  └─────────┘  │
└ ─ ─ ─ ─ ─ ─ ─ ┘
```

# FIG. 8B

```
┌ ─ ─ ─ ─ ─ ─ ─ ─ ─ ─ ─ ─ ─ ─ ─ ┐
                            438
│  ┌──────────────────────────┐  │
   │  Spatial Gradients   526 │
│  └──────────────────────────┘  │
               │
│              ▼                 │
   ┌──────────────────────────┐
│  │  Hysteresis Threshold    │  │
   │                     530  │
│  └──────────────────────────┘  │
               │
│              ▼                 │
   ┌──────────────────────────┐
│  │ Region-of-Interest (ROI) Selection │
   │                     534  │
│  └──────────────────────────┘  │
               │
│              ▼                 │
   ┌──────────────────────────┐
│  │  Matching Edges Filter   │  │
   │                     538  │
│  └──────────────────────────┘  │
└ ─ ─ ─ ─ ─ ─ ─ ─ ─ ─ ─ ─ ─ ─ ─ ┘
```

# FIG. 8C

Close to obtain Apparent Amniotic Fluid Area
(AAFA) or Volume (AAFV)          546

↓

Open to reduce AAFA or AAFV          550

↓

Remove Deep Regions to further reduce
AAFA or AAFV          554

↓

Remove Fetal Head Regions to further reduce
AAFA or AAFV          560

464

# FIG. 8D

```
┌ ─ ─ ─ ─ ─ ─ ─ ─ ─ ─ ─ ─ ─ ─ ─ ─ ─ ┐
│        ┌─────────────────────┐      560 │
│        │  Gestational Age     │          │
│        │              726     │          │
│        └─────────────────────┘          │
│                  │                       │
│                  ▼                       │
│        ┌─────────────────────┐          │
│        │ Determine Head Diameter Factor │
│        │              730     │          │
│        └─────────────────────┘          │
│                  │                       │
│                  ▼                       │
│        ┌─────────────────────┐          │
│        │  Head Edge Detection │          │
│        │              734     │          │
│        └─────────────────────┘          │
│                  │                       │
│                  ▼                       │
│        ┌─────────────────────┐          │
│        │  Hough Transform     │          │
│        │              736     │          │
│        └─────────────────────┘          │
└ ─ ─ ─ ─ ─ ─ ─ ─ ─ ─ ─ ─ ─ ─ ─ ─ ─ ┘
```

# FIG. 8E

```
┌─ ─ ── ── ── ── · ── ── ── ── ── ── ─┐
│                                          736 │
│   ┌──────────────────────────────┐          │
│ ·· │                              │          │
│    │   Polar Hough Transform      │          │
│    │                         738  │          │
│    └──────────────────────────────┘          │
│                   │                   -      │
│                   ▼                          │
│   ┌───────────────────────────────────────┐  │
│   │                                       │  │
│   │     Find Maximum Hough Value          │  │
│   │                              742      │  │
│   └───────────────────────────────────────┘  │
│                   │                          │
│                   ▼                          │
│   ┌───────────────────────────────────────┐  │
│   │                                       │  │
│   │        Fill Circle Region             │  │
│   │                              746      │  │
│   └───────────────────────────────────────┘  │
│                                              │
└─ ── ── ── ── ── ── ── ── ── ── ── ── ─┘
```

# FIG. 8F

**FIG. 9**

820

840

850

858

862

864

# FIG. 10

**FIG. 11**

# FIG. 12

Sonographer's
Outline →

Algorithm's
Outline →

# FIG. 13

Input Image — 1020

↓

Heat and Shock Filtering Algorithm — 1030

↓

Spatial Gradient Algorithm — 1034

1000

Point-Positioning Algorithm — 1038

Previous Image Contour Input — 1046

1042

Optimal Path Algorithm

↓

Compute Volume Algorithm — 1048

↓

Output Volume — 1052

FIG. 14

1204    1206    1208    1210    1212

# FIG. 15

1222          1226          1230

EP 1 538 986 B1

# FIG. 16A

FIG. 16B  Heat and shock filtering of blurred noisy signal

Legend:
— original signal
-·- noisy signal
cleaned by heat flow
······ shock filtered heat flow

# FIG. 17

FIG. 18

FIG. 19

FIG. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6346124 B, Geiser
- US 6213949 B, Ganguly
- US 5235985 A, McMorrow
- US 5605155 A, Chalana
- US 6375616 B, Soferman
- US 5588435 A, Weng
- US 20020102023 A1
- US 5644513 A, Rudin

**Non-patent literature cited in the description**

- **Phelan JP ; Smith CV ; Broussard P ; Small M.** Amniotic fluid volume assessment with the four-quadrant technique at 36-42 weeks' gestation. *J Reprod Med,* July 1987, vol. 32 (7), 540-2
- **Moore TR ; Cayle JE.** The amniotic fluid index in normal human pregnancy. *Am J Obstet Gynecol,* May 1990, vol. 162 (5), 1168-73
- **Sepulveda W ; Flack NJ ; Fisk NM.** Direct volume measurement at midtrimester amnioinfusion in relation to ultrasonographic indexes of amniotic fluid volume. *Am J Obstet Gynecol,* April 1994, vol. 170 (4), 1160-3
- **Magann EF ; Perry KG Jr ; Chauhan SP ; Anfanger PJ ; Whitworth NS ; Morrison JC.** The accuracy of ultrasound evaluation of amniotic fluid volume in singleton pregnancies: the effect of operator experience and ultrasound interpretative technique. *J Clin Ultrasound,* June 1997, vol. 25 (5), 249-53
- **Segiv C ; Akselrod S ; Tepper R.** Application of a semiautomatic boundary detection algorithm for the assessment of amniotic fluid quantity from ultrasound images. *Ultrasound Med Biol,* May 1999, vol. 25 (4), 515-26
- **Grover J ; Mentakis EA ; Ross MG.** Three-dimensional method for determination of amniotic fluid volume in intrauterine pockets. *Obstet Gynecol,* December 1997, vol. 90 (6), 1007-10
- **M. Kass ; A. Witkin ; D. Terzopolous.** Snakes : Active Contour Models. *International Journal of Computer Vision,* 1988, 321-331
- **A.X. Falacao ; J.K. Udupa ; S. Samarasekara ; S. Sharma.** *Graphical Models and Image Processing,* 1998, vol. 60, 233-26
- **E.W. Dijkstra.** A note on two problems in connection with graphs. *Numerical Math,* 1959, vol. 1, 269-271
- **P. Perona ; J. Malik.** Scale-space and edge detection using aniostropic diffusion. *IEEE Trans. Pattern Analysis and Machine Intelligence,* 12 July 1990, 629-639
- **J.A. Sethian.** Level Set Methods and Fast Marching Methods. Cambridge University Press, 1999
- **S. Osher ; L.I. Rudin.** Feature-oriented image enhancement using shock filters. *SIAM Journal of Numerical Analysis,* August 1990, vol. 27, 919-940